(19) Europäisches Patentamt
European Patent Office
Office européen des brevets

(11) Numéro de publication: **0 288 395**
**A2**

(12) **DEMANDE DE BREVET EUROPEEN**

(21) Numéro de dépôt: **88401000.0**

(22) Date de dépôt: **22.04.88**

(51) Int. Cl.⁴: **C 07 H 15/04**
A 61 K 31/70

(30) Priorité: **22.04.87 FR 8705709**
**12.01.88 FR 8800285**

(43) Date de publication de la demande:
**26.10.88 Bulletin 88/43**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Roger, Pierre**
**6, rue Paul Valéry**
**F-78423 Montigny-les-Bretonneux (FR)**

**Fournier, Jean-Paul**
**7, rue La Condamine**
**F-75017 Paris (FR)**

**Martin, Alain**
**20, Route de Daigny Givonne**
**F-08200 Sedan (FR)**

**Monneret, Claude**
**9, rue Lamoricière**
**F-75012 Paris (FR)**

(74) Mandataire: **Grosset-Fournier, Chantal Catherine et al**
**SC Ernest Gutmann/Yves Plasseraud 67 boulevard Haussmann**
**F-75008 Paris (FR)**

La demande, qui etait incomplète au moment du dêpot, est publiée telle quelle (article 93 (2) CBE). Le passage de la description ou des revendications qui comporte manifestement une omission sur les pages 34 et 35 du texte original, est présenté comme tel. Revendications pour les Etats contractants suivants: ES + GR.

(54) **Dérivés de nitrosourées, leur nouveau procédé de préparation et leurs applications thérapeutique.**

(57) L'invention a pour objet de nouvelles désoxy-4 sucre nitrosourées répondant à la formule suivante

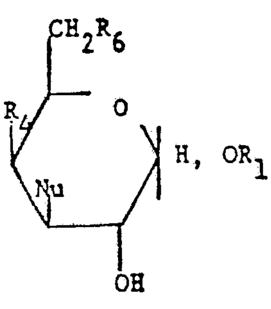

I

sous forme de l'un des deux anomères α ou β,
dans laquelle
- $R_1$ représente notamment un groupe alcoyle de 1 à 12 atomes de carbone,
- $R_4$ représente notamment H,
- $R_6$ représente notamment OH,
- Nu représente le groupe $-\underset{\underset{O}{\|}}{C}-\underset{\underset{NO}{|}}{N}-CH_2CH_2Hal'$, Hal' représentant notamment le chlore.

Ces composés sont utiles pour la préparation de médicaments.

EP 0 288 395 A2

**Description**

## DERIVES DE NITROSOUREES, LEUR NOUVEAU PROCEDE DE PREPARATION ET LEURS APPLICATIONS THERAPEUTIQUES

La présente invention est relative à de nouveaux dérivés de nitrosourées, et plus particulièrement à des désoxy-4 sucres nitrosourées, à leur procédé de préparation et à leurs applications en tant que substances thérapeutiquement actives.

On a déjà décrit des dérivés de désoxy-2 sucres nitrosourées et de désoxy-4 sucres nitrosourées dans la demande de brevet français n° 83 13878 du 30 août 1983 (publié sous le n° 2 551 068), ces composés présentant d'intéressantes propriétés thérapeutiques, notamment antitumorales.

Plus précisément, la susdite demande française décrit des composés de formule A :

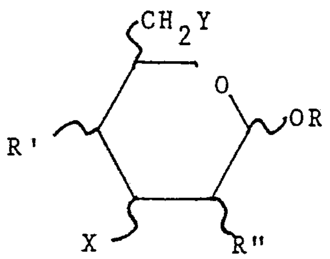

A

dans laquelle

- R représente un atome d'hydrogène, un groupe alcoyle de 1 à 30, de préférence de 1 à 12 atomes de carbone ou un groupe aralçoyle de 7 à 12, de préfére, ce de 7 à 9 atomes de carbone, éventuellement substitué par un plusieurs, notamment jusqu'à 3, atomes d'halogène, groupes $NO_2$, $NH_2$, $CF_3$ ou groupes alcoxy de 1 à 4 atomes de carbone,
- X représente un groupe hydroxy ou un groupe $NR_1R_2$
- Y représente un atome d'hydrogène
  un groupe hydroxy ou un groupe

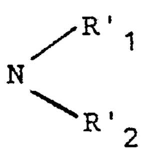

où $R_1$ et/ou $R'_1$ représentent chacun un atome d'hydrogène ou un groupe $-\underset{\underset{O}{\|}}{C}-\underset{\underset{NO}{|}}{N}-$ $CH_2CH_2Hal$, Hal étant un halogène, de

préférence Cl,

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs, notamment jusqu'à 3, atomes d'halogène, groupes $NO_2$, $NH_2$, $CF_3$ ou groupes alcoxy de 1 à 4 atomes de carbone,

- R′ ou R″ représentent l'hydrogène, OH, OM, M représentant un groupe alcoyle comprenant de 1 à 30, de préférence de 1 à 12 atomes de carbone, un group aryle de 4 à 10 atomes de carbone, un groupe aralcoyle comprenant de 7 à 12, de préférence de 7 à 9 atomes de carbone, les groupes aryle et aralcoyle étant éventuellement substitués par un ou plusieurs, notamment jusqu'à 3, atomes d'halogène, groupes $NO_2$, $NH_2$, $CF_3$ ou groupes alcoxy de 1 à 4 atomes de carbone, ou M pouvant représenter également un groupe acyle de 2 à 8 atomes de carbone, de préférence 2 ou 3, ou un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone

sous réserve qu'au moins X représente

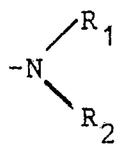 , avec $R_1$

avec $R_1$ représentant - $\underset{\| \atop O}{C}-\underset{| \atop NO}{N}-$ $CH_2CH_2Hal$ ou Y représente

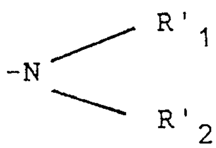

avec $R'_1$ représentant - $\underset{\| \atop O}{C}-\underset{| \atop NO}{N}-$ $CH_2CH_2Hal$

et sous réserve que soit R′ soit R″ représente l'hydrogène,

. R′ et R″ ne pouvant pas représenter simultanément l'hydrogène.

Ces composés sont obtenus à partir du composé de formule Abis :

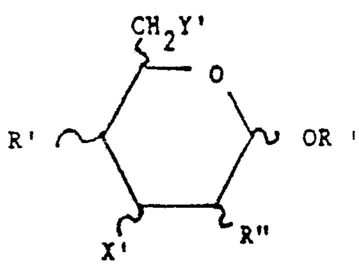

Abis

das laquelle :

- X′ représente OH ou $NHR_2$
- Y′ représente H, OH ou $NHR'_2$
- R, R′, R″, $R_2$ et $R'_2$ ont les significations indiquées à propos de la formule A et l'un au moins des groupes X′ et Y′ représente $NHR_2$ ou $NHR'_2$,

1) lequel est soumis à l'action d'isocyanate d'halogéno-2-éthyle pour transformer le groupe $NHR_2$ ou $NHR'_2$ du composé de formule Abis, respectivement en $NR_2\underset{\| \atop O}{C}NHCH_2CH_2Hal$ ou $NR'_2\underset{\| \atop O}{C}NHCH_2CH_2Hal$; Hal représentant un atome d'halogène.

2) le composé obtenu à l'étape précédente est soumis à une nitrosation à l'aide de nitrite d'un métal alcalin pour transformer les groupes $NR_2\underset{\| \atop O}{C}NHCH_2CH_2Hal$ ou $NR'_2\underset{\| \atop O}{C}NHCH_2CH_2Hal$ respectivement en $NR_2\ \underset{\| \atop O}{C}\underset{| \atop NO}{N}\ \ H_2CH_2Hal$ ou $NR'_2\ \underset{\| \atop O}{C}\underset{| \atop NO}{N}\ \ H_2CH_2Hal$.

Les composés de formule Abis sont obtenus à partir de composés intermédiaires comportant un groupe amine primaire en position 3 et/ou en position 6, de la façon qui peut être illustrée comme suit :

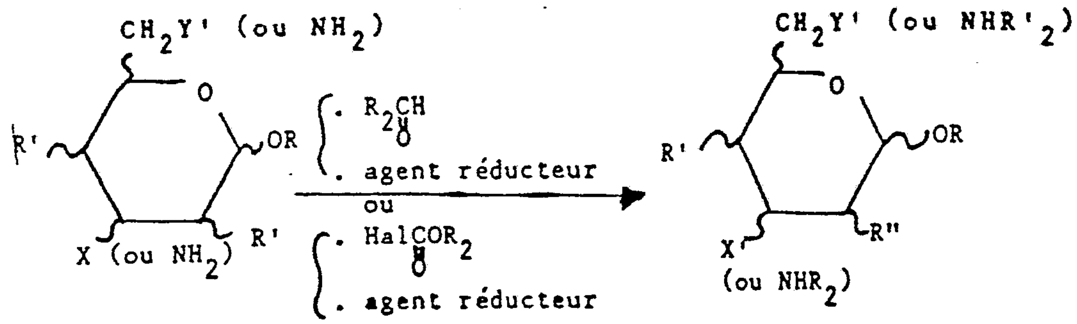


Dans le cas des composés désoxy-2 de configuration D, ces composés intermédiaires comportant l'amine primaire peuvent être obtenus à partir d'azide comportant la fonction désoxy-2 et un hydrogène en position 2.

Dans le cas des désoxy-2 de configuration β-L, les composés comportant l'amine primaire peuvent être obtenus à partir de l'azide comportant la fonction désoxy-2 et un hydrogène en position 2.

Les composés désoxy-4 dans lesquels Y représente H peuvent également être obtenus par des procédés équivalents, à partir soit de l'azide comportant la fonction désoxy-4 avec un hydrogène en position 4, par exemple pour les composés désoxy-4 de configuration β-L, soit à partir d'un composé intermédiaire comportant un groupe amine primaire par exemple pour les composés désoxy-4 de configuration α-D.

Or, il s'est avéré que l'accès à certaines nitrosourées ne pouvait être envisagé que par d'autres voies, surtout au niveau des synthèses des produits de départ ou des produits intermédiaires. Il en est particulièrement ainsi des composés de formule A dans laquelle R′ représente l'hydrogène et Y représente OH, pour lesquels les composés à fonctions amine ou azide comportant la fonction désoxy-4 et un hydrogène en position 4 ne sont pas accessibles à ce jour.

Cette particularité fait des composés désoxy-4 à configuration D un cas à part et, par conséquent, une classe originale de composés auxquels la présente invention donne désormais accès. Cela est d'autant plus remarquable

- que certains des membres de cette classe particulière de composés présentent des activités antitumorales encore jamais atteintes dans des essais pharmacologiques considérés comme particulièrement significatifs à ce jour ;
- que le procédé ci-après décrit étend l'accès de l'homme du métier à d'autres catégories de produits, parmi lesquels certains présentent des activités antitumorales aussi remarquables : il s'agit notamment des composés dans lesquels le carbone en position 4 peut être substitué par un atome d'halogène et/ou le carbone en position 6 peut être substitué par un groupe acyle.

Les nouveaux composés de l'invention répondent à la formule I suivante :

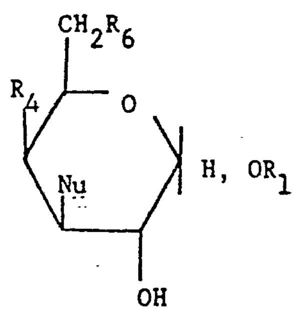

sous forme de l'un des deux anomères α ou β,

dans laquelle

- $R_1$ représente un groupe alcoyle de 1 à 12 atomes de carbone, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente H ou Hal, Hal étant un atome de Cl ou Br, notamment Cl,
- $R_6$ représente OH ou $O-\underset{\underset{O}{\|}}{C}-R$,

  R représentant un groupe alcoyle de 1 à 6 atomes de carbone, un groupe aryle non substitué ou substitué sur le noyau aromatique par un ou plusieurs atomes, notamment jusqu'à 3 atomes

d'halogène, par un à trois groupes $NO_2$, $CF_3$ ou groupes alcoxy de 1 à 4 atomes de carbone,

- Nu représente le groupe $NH-\underset{\underset{O}{\|}}{C}-\underset{\underset{NO}{|}}{N}-CH_2CH_2Hal'$, Hal' représentant un halogène, choisi parmi F, Cl, Br et I, identique ou différent de Hal et notamment le chlore.

On a constaté

- que les nouveaux produits ainsi préparés présentent un meilleur indice thérapeutique que celui de de l'ensemble des composés connus de façon générale et globale, ce qui rend ces produits non équivalents à ceux de la famille à laquelle ils appartiennent ;
- que les nouveaux composés de désoxy-4 sucres présentent des propriétés physicochimiques facilitant leur utilisation thérapeutique ;
- que les nouveaux composés de désoxy-4 sucres nitrosourées ont l'avantage de se présenter sous une forme solide et stable ;
- que les nouveaux dérivés de désoxy-4 sucres nitrosourées sont remarquablement actifs vis-à-vis de tumeurs généralement très difficiles à combattre, et en particulier permettant la régression totale de certains types de tumeurs ;
- que les nouveaux composés sont thérapeutiquement actifs, et que leur l'activité se révèle être aussi efficace lorsque ces nouveaux composés sont administrés par voie intrapéritonéale chez l'animal que lorsqu'ils sont administrés par voie intraveineuse, ce qui n'est pas habituellement le cas pour ce type de substances et est prédictif d'une activité chez l'homme.

Dans la formule I ci-dessus définie et certaines des formules représentées ci-après, on a représenté la liaison entre l'atome d'hydrogène et le groupe $OR_1$, en position 1 sur le carbone du cycle, par le symbole | $H,OR_1$.

Cette représentation signifie que le groupe $OR_1$, peut être soit en position α, soit en position β, selon la représentation de HAYWORTH.

Dans la suite de la description, le terme alcoyle inclut les alcoyles linéaires, ramifiés ou cycliques (cycloalcoyle).

Les composés selon l'invention de formule I sont des didésoxy-3,4 α-D-xylohexopyrannosides, des didésoxy-3,4 β-D-xylohexopyrannosides, des didésoxy-3,4 α-D-galactohexopyrannosides et des didésoxy-3,4 β-D-galactohexopyrannosides

Une classe avantageuse de composés selon l'invention est constituée par les dérivés de nitrosourées répondant à la formule I ci-dessus et dans laquelle

- $R_1$ représente un groupe alcoyle de 1 à 12 atomes de carbone,
- $R_4$ représente un atome d'halogène,
- $R_6$ représente OH ou $O-\underset{\underset{O}{\|}}{C}-R$, R ayant les significations indiquées ci-dessus.

Dans cette classe de dérivés de nitrosourées selon l'invention, sont particulièrement avantageux les composés de formule I indiquée ci-dessus dans laquelle

- $R_1$ représente un groupe $CH_3$,
- $R_4$ représente un atome d'halogène,
- $R_6$ représente OH ou $O-\underset{\underset{O}{\|}}{C}-R$,

R ayant les significations indiquées ci-dessus.

Dans cette même classe de dérivés de nitrosourées selon l'invention sont particulièrement avantageux les composés de formule I indiquée ci-dessus dans laquelle d'une part

- $R_1$ représente un groupe $CH_3$,
- $R_4$ représente Cl,
- $R_6$ représente OH ou $O-\underset{\underset{O}{\|}}{C}-R$, R ayant les significations

et d'autre part

- $R_1$ représente un groupe $CH_3$,
- $R_4$ représente un halogène,
- $R_6$ représente $O-\underset{\underset{O}{\|}}{C}-R$, R ayant les significations indiquées ci-dessus.

Une autre classe avantageuse de dérivés de nitrosourées selon l'invention est constituée par ceux de formule I indiquée ci-dessus dans laquelle

- $R_1$ représente un groupe $CH_3$,
- $R_4$ représente l'hydrogène,
- $R_6$ représente OH.

Une autre classe avantageuse de dérivés de nitrosourées selon l'invention est constituée par ceux de formule I ci-dessus indiquée dans laquelle

- $R_1$ représente un groupe $CH_3$,
- $R_4$ représente l'hydrogène,
- $R_6$ représente $O-\underset{\underset{O}{\|}}{C}-R$, R ayant les significations

indiquées ci-dessus.

Parmi les produits cités ci-dessus, les plus intéressants sont ceux ayant les formules suivantes :

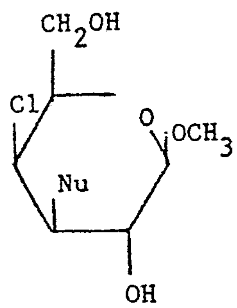

1803

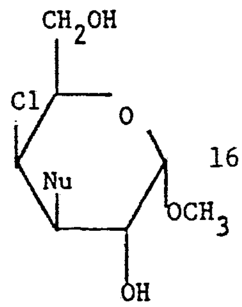

1676

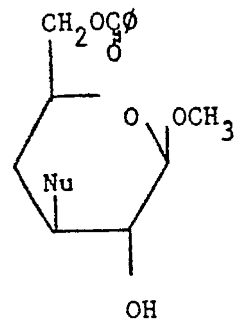

1674

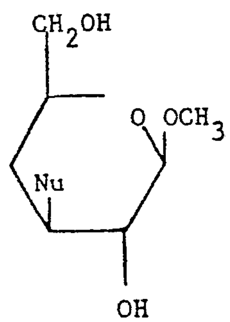

1675

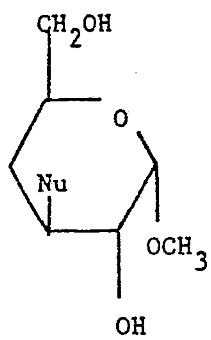

1677

La présente invention a également pour objet un procédé de préparation des nouveaux dérivés de formule I,

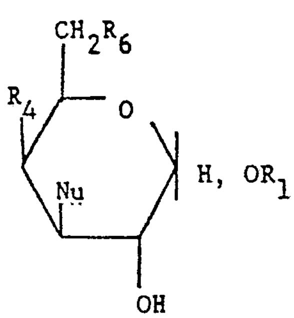

I

sous forme de l'un des deux anomères α ou β,

dans laquelle

- $R_1$ représente un groupe alcoyle de 1 à 12 atomes, ou un groupe aralcoyle de 7 à 12, de préférence de 7 à 9 atomes de carbone, éventuellement substitué par un ou plusieurs, notamment jusqu'à 3, atomes d'halogène,

par un à trois groupes $NO_2$, $CF_3$ ou groupes alcoxy de 1 à 4 atomes de carbone,
- $R_4$ représente Hal, Hal étant un atome d'halogène, notamment Cl,
- $R_6$ représente OH ou $O-\underset{\underset{O}{\|}}{C}-R$, R ayant les significations indiquées ci-dessus.
- Nu représente le groupe $NH-\underset{\underset{O}{\|}}{C}-\underset{\underset{NO}{|}}{N}-CH_2-CH_2-Hal'$, Hal' étant identique ou différent de Hal et représentant un halogène, notamment le chlore, caractérisé en ce que

a) dans une première série de réactions, on fait réagir le composé de formule II

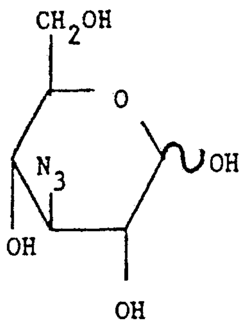

II

sous forme de mélange des anomères α et β avec un agent de glycosylation, constitué par un alcool $R_1OH$, $R_1$ ayant la signification indiquée ci-dessus, pour transformer le groupe OH en position 1, en groupe $OR_1$,

on traite le produit ainsi obtenu avec un dérivé fonctionnel de l'acide R-COOH pour transformer le groupe OH en position 6, en groupe $O-\underset{\underset{O}{\|}}{C}-R$,

* on sépare les anomères α et β par une méthode appropriée, et
* on fait réagir chacun des anomères α ou β avec un agent d'halogénation pour introduire un halogène en position 4,

le composé obtenu étant sous forme de l'un des anomères α ou β et répondant à la formule III

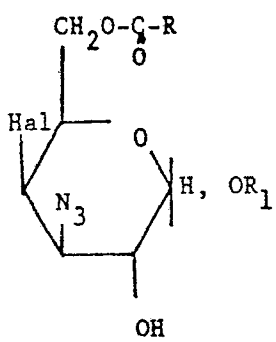

III

dans laquelle
- R et $R_1$ ont les significations indiquées dans la formule I,
- Hal représente un halogène,

b) ensuite, dans une deuxième étape, on effectue la réduction du composé de formule III, pour réduire $N_3$ en $NH_2$, cette réduction étant en outre effectuée dans des conditions telles que l'halogène en position 4 soit ou non hydrogénolysé, cette réduction étant éventuellement suivie de l'hydrolyse du groupe $O-\underset{\underset{O}{\|}}{C}-R$ en position 6 pour donner un reste OH,

le composé obtenu à l'issue de cette deuxième étape étant sous forme de l'un des anomères α ou β, et répondant à la formule IV suivante :

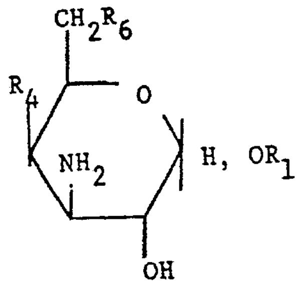

IV

dans laquelle
$R_1$, $R_4$, $R_6$ ont les significations indiquées dans la formule I,
c) dans une troisième série de réactions, on effectue la réaction des anomères α ou β de formule IV sur un isocyanate d'halogéno éthyle pour transformer $NH_2$ en $NH\underset{\underset{O}{\|}}{C}NHCH_2CH_2Hal'$ et on traite le produit ainsi obtenu par

un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,
afin d'obtenir le composé de formule I, sous forme de l'un des anomères α ou β.

Dans la formule II et certaines des formules ci-après, on a représenté la liaison entre OH d'une part et le carbone en position 2 de la structure cyclique d'autre part, par le symbole ∿ . Cette représentation signifie que le groupe OH peut être en position α, ou en position β.

Comme dérivé fonctionnel de l'acide R-COOH, on utilise les dérivés d'acide habituellement utilisés pour l'estérification d'hydroxyles primaires, tel qu'un halogénure, de préférence le chlorure, ou un anhydride.

Comme agent d'halogénation, on utilise de préférence le chlorure ou le bromure de sulfuryle.

Le composé de départ de formule II

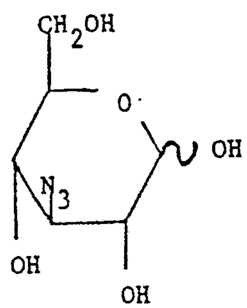

II

sous forme du mélange des anomères α et β est décrit dans la littérature (notamment par Redlich dans Liebigs Ann. Chem. 1981, p. 1215 à 1222).

Un mode de réalisation préféré du procédé de l'invention pour la préparation des dérivés de nitrosourées de formule I comprend
- la réaction du composé de formule II ci-dessus sous forme de mélange des anomères α et β avec un agent de glycosylation constitué par $R_1OH$, $R_1$ ayant la signification indiquée ci-dessus, pour donner le composé de formule V

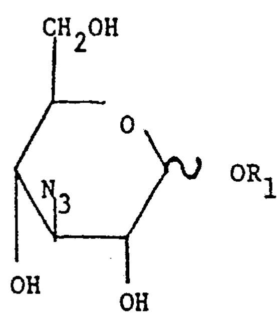

V

- l'introduction d'un groupe acyloxy en position 6, de formule $O-\underset{\overset{\|}{O}}{C}-R$, R ayant la signification indiquée ci-dessus, pour donner le composé de formule

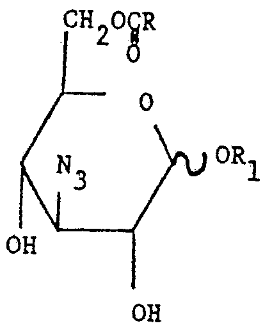

VI

- la séparation du composé VI en deux anomères α et β de formule VII

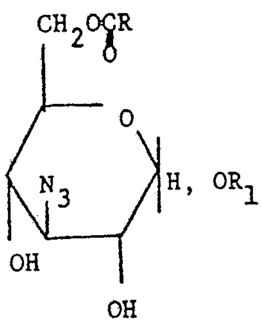

VII

- la réaction des anomères α ou β, de formule VII, avec un agent d'halogénation, pour donner les anomères α ou β de formule III

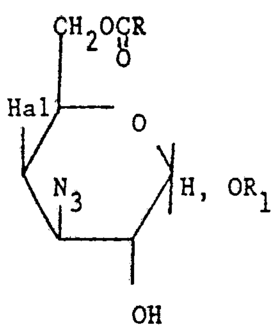

III

. puis

* soit la réduction des anomères α ou β de formule III ainsi obtenus, dans des conditions telles que $N_3$ soit réduit en $NH_2$ sans hydrogénolyse de l'halogène en position 4, pour donner les anomères α ou β de formule IX

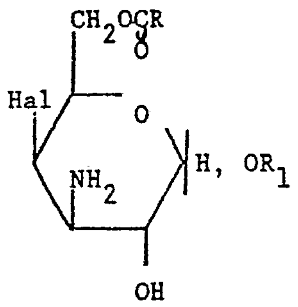

IX

cette réduction étant

** ou bien suivie de la réaction des anomères α ou β de formule IX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$, afin d'obtenir les composés de formule I, dans lesquels $R^4$ représente un halogène et $R_6$ représente $O-\underset{\underset{O}{\|}}{C}-R$,

** ou bien suivie de l'hydrolyse à partir des anomères α ou β de formule IX, du groupe $O-\underset{\underset{O}{\|}}{C}-R$, en position 6, et la formation d'un reste OH, puis la réaction des anomères α ou β de formule IX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$ pour donner les composés de formule I, dans lesquels

$R_4$ représente un halogène et

$R_6$ représente OH,

. ou

* soit la réduction respectivement des anomères III dans des conditions telles que l'halogène en position 4 soit remplacé par H et le groupe $N_3$ soit réduit en $NH_2$, pour donner les anomères α ou β de formule X

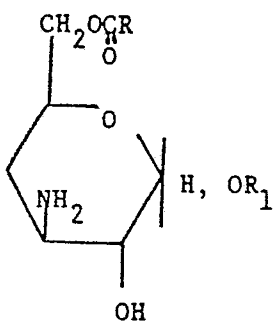

X

cette réduction étant suivie

** ou bien de la réaction des anomères α ou β de formule X sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

respectivement pour obtenir les composés de formule I dans lesquels
$R_4$ représente H,
$R_6$ représente $O-\underset{\underset{O}{\|}}{C}-R$

** ou bien de l'hydrolyse à partir des anomères α ou β de formule X du groupe $O-\underset{\underset{O}{\|}}{C}-R$ en position 6 et pour donner un

reste OH pour donner des anomères α ou β de formule XI

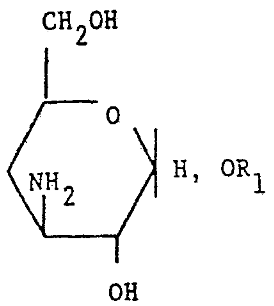

XI

suivi de la réaction des anomères α ou β de formule XI sur un isocyanate d'halogéno éthyle pour transformer $NH_2$ en $NH\underset{\underset{O}{\|}}{C}NHCH_2CH_2Hal'$ et de l'action d'un

nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

pour obtenir des anomères α ou β de formule I dans lesquels $R_4$ représente H, et $R_6$ représente OH.

Selon un mode de réalisation avantageux du procédé de l'invention d'obtention des dérivés de formule I, la réduction des composés de formule III telle que $N_3$ soit réduit en $NH_2$ et Hal soit remplacé par l'hydrogène, est effectuée à l'aide d'hydrure de tributylétain, en présence d'azo 2-2′ bis isobutyronitrile.

De façon avantageuse, après la glycosylation du composé de formule II, le dérivé fonctionnel de l'acide RCOOH mis en réaction avec le composé de formule II pour introduire en position 6 un groupement benzoyle, est le chlorure de benzoyle en présence de l'oxyde de bis tributylétain.

Un autre mode de réalisation préféré du procédé de l'invention pour la préparation de dérivés de nitrosourées de formule I dans laquelle $R_1$, $R_4$, $R_6$ et Nu ont les significations indiquées ci-dessus, comprend

- la glycosylation du composé de formule II sous forme du mélange d'anomères α et β à l'aide de $CH_3OH$, pour obtenir le composé de formule XII

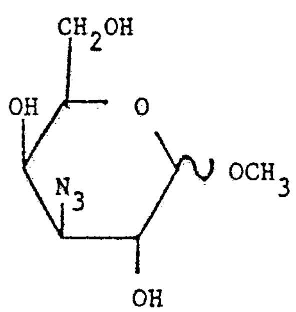

XII

- la réaction du composé de formule XII précédemment obtenu avec du chlorure de benzoyle et de l'oxyde de tributylétain pour obtenir le composé de formule XIII

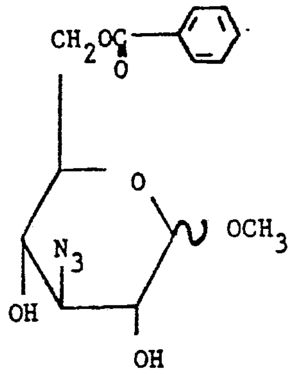

XIII

- la séparation des deux anomères α et β à partir du composé de formule XIII
- la réaction de l'un ou l'autre des anomères α ou β avec $SO_2Cl_2$, pour obtenir les anomères α ou β de formule XIV

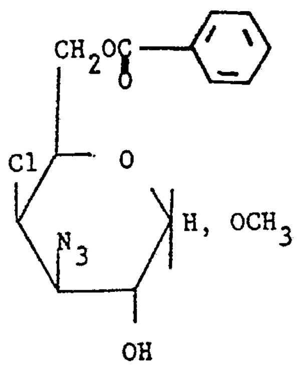

XIV

et

. soit l'hydrogénation catalytique des anomères α ou β de formule XIV précédemment obtenus pour donner les anomères α ou β de formule XV

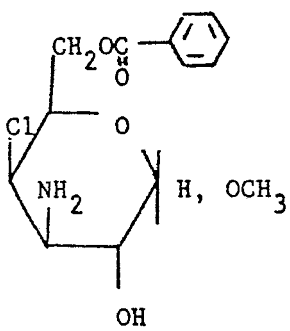

XV

suivi

.. soit de la réaction des anomères α ou β de formule XV sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

et pour donner les anomères α ou β de formule

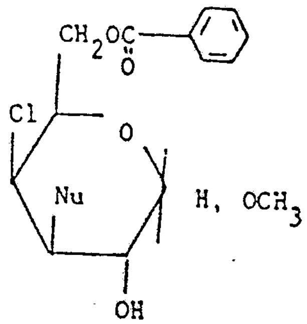

XVI

.. soit de la réaction sur les anomères α ou β de formule XV avec une base, notamment un alcoolate alcalin, pour hydrolyser le groupe benzoyle et donner les anomères α ou β de formule XVII

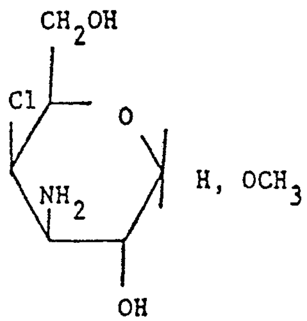

XVII

. suivi de la réaction des anomères α ou β de formule XVII sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

et pour donner les anomères α ou β de formule XVIII

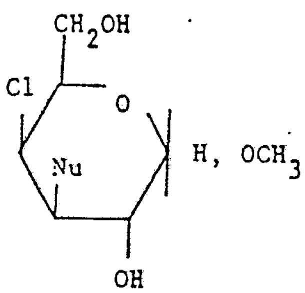

XVIII

. soit la réduction des anomères α ou β de formule XIV, à l'aide d'hydrure de tributylétain, en présence d'azo 2-2' bis isobutyronitrile, pour donner les anomères α ou β de formule

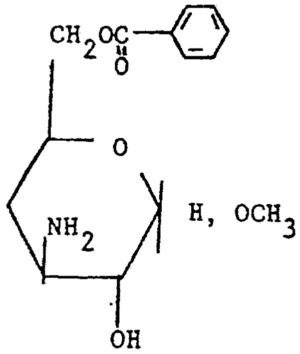

XIX

suivi

.. soit de la réaction des anomères α ou β de formule XIX sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium, pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

et pour donner les anomères α ou β de formule XX

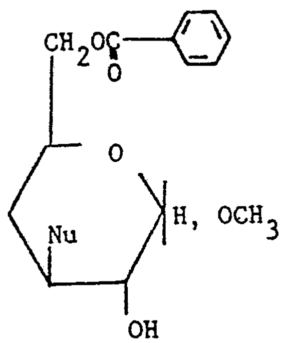

XX

.. soit de la réaction des anomères α ou β de formule XIX avec une base, notamment un alcoolate alcalin, pour hydrolyser le groupe benzoyle et donner les anomères α ou β de formule XXI

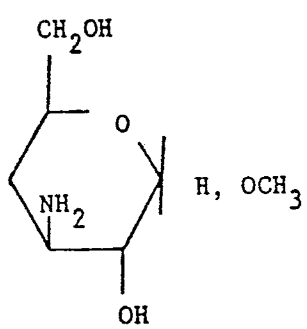

XXI

suivi de la réaction des anomères α ou β de formule XXI sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$

et pour donner les anomères α ou β de formule XXII

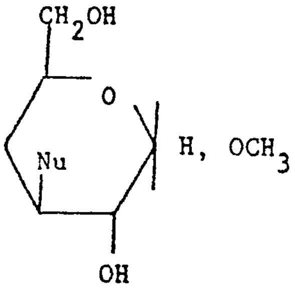

XXII

Dans la mise en oeuvre du procédé de l'invention, les composés de formule III

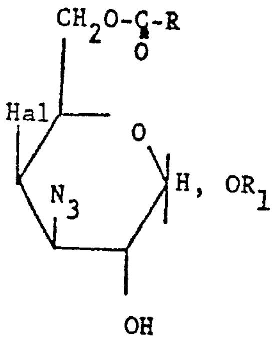

III

dans laquelle
- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- R représente un groupe alcoyle de 1 à 6 atomes de carbone, un groupe aryle non substitué ou substitué sur le noyau aromatique par un ou plusieurs atomes, notamment jusqu'à 3 atomes d'halogène, groupes $NO_2$, $CF_3$ ou groupes alcoxy de 1 à 4 atomes de carbone,
- Hal représente un halogène, notamment le chlore, sont nouveaux, sous forme d'un de leurs anomères α ou β.

Parmi les composés de formule III, un groupe de composés préférés est constitué par ceux de formule III dans laquelle
- $R_1$ représente un groupe alcoyle de 1 à 12 atomes de carbone,
- R représente

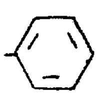

Un nouveau composé particulièrement avantageux répond à la formule suivante

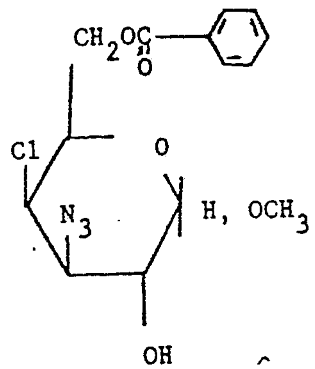

IIIa

sous forme de l'anomère α ou β.

Les composés de formule III, notamment les deux composés de formule IIIa (anomère α ou anomère β), sont les intermédiaires-clé du procédé et représentent un autre aspect de la présente invention.

Outre les dispositions qui précèdent, l'invention comprend encore d'autres dispositions, qui ressortiront de la description qui va suivre.

L'invention vise non seulement les nouveaux dérivés de nitrosourées conformes aux dispositions qui précèdent et leur procédé de préparation, mais aussi les nouveaux dérivés de nitrosourées en tant que substance thérapeutiquement active, ainsi que les compositions, en particulier des compositions thérapeutiques dans lesquelles entrent lesdits dérivés.

L'invention sera mieux comprise à l'aide du complément de description qui va suivre, qui se réfère à des exemples de mise en oeuvre du procédé objet de la présente invention.

Il doit être bien entendu, toutefois, que ces exemples de mise en oeuvre sont donnés uniquement à titre d'illustration de l'objet de l'invention, dont ils ne constituent en aucune manière une limitation.

EXEMPLE 1 :

Préparation du [(chloro-2 éthyl)-3 nitroso-3 ureido]-3 didésoxy-3,4 β-D-xylo-hexopyrannoside de méthyle (IC. 1675)

1) Préparation de l'azido-3 benzoyl-6 désoxy-3 β-D-gluco-hexopyrannoside de méthyle

29 g d'azido-3 désoxy-3 D gluco-hexopyrannoside sont mis en solution dans 600 ml de méthanol chlorhydrique N puis portés au reflux 2 heures puis évaporés à sec sous vide.

Le produit est repris par du chlorure de méthylène puis lavé à l'eau jusqu'à absence d'ion chlorure. La phase organique séchée sur sulfate de sodium puis évaporée à sec sous vide donne un résidu de 31 g de produit glycosyle utilisé tel quel dans l'étape suivante.

Le produit est dissout dans 1 250 ml de toluène anhydre. On ajoute alors 90 ml d'oxyde de bis tributylétain et on porte 3 heures au reflux. On ramène le milieu réactionnel à - 15° et on additionne goutte à goutte 40 ml de chlorure de benzoyle dilué dans 200 ml de chlorure de méthylène. Après 24 heures d'agitation, le milieu réactionnel est évaporé à sec sous vide puis chromatographié sur silice (éluant $CH_2Cl_2CH_3OH$ 98:2).

On obtient ainsi sous forme de laque :

- 7,5 g d'azido-3 benzoyl-6 désoxy-3β-D gluco-hexopyrannoside de méthyle.
  $[\alpha]_D$ = - 3,5° (c 0,83 $CHCl_3$)
- 7,0 g d'azido-3 benzoyl-6 déoxy-3 α-D gluco-hexopyrannoside de méthyle.
  $[\alpha]_D$ = + 102,0° (c 1,14 $CH_2Cl_2$).

2) Préparation de l'azido-3 benzoyl-6 chloro-4 didésoxy-3,4 β-D-galacto-hexopyrannoside de méthyle

A 4,8 g (0,015 mole) de l'azido-3 benzoyl-6 déoxy-3 β-D-gluco-hexopyrannoside de méthyle dans 250 ml de pyridine anhydre, on ajoute à 0°C et goutte à goutte, 12,5 ml (0,15 mole) de chlorure de sulfuryle. La solution est agitée pendant 18 heures à 0°C puis ramenée à température ambiante. Le milieu réactionnel est ensuite versé sur 500 g de glace, extrait par du dichlorométhane.

La phase organique, lavée à l'acide sulfurique N, puis à l'eau est séchée sur sulfate de sodium, filtrée et évaporée. Le résidu huileux, purifié par chromatographie sur silice éluant hexane-acétate d'éthyle 4:1, donne 4,05 g (80 %) de cristaux blancs.

$C_{14}H_{16}ClN_3O_5$ : 341,7
F : 84-86° (hexane-acétate d'éthyle)
$[\alpha]_D$ : + 2,3° (c 0,88 $CHCl_3$)

3) Préparation de l'amino-3 benzoyl-6 didésoxy-3,4 β-D-xylo-hexopyrannoside de méthyle

A 4,05 g (0,012 mole) du composé précédent dans 200 ml de toluène anhydre, est ajouté de l'azobisisobutyronitrile (700 mg, 4,27 mmoles), puis sous azote, goutte à goutte, de l'hydrure de tributylétain

(12,6 ml, 47 mmoles). Le milieu réactionnel est porté au reflux pendant 2 heures puis évaporé sous pression réduite.

Par chromatographie sur silice éluant dichlorométhane-méthanol ammoniacal 19:1; on isole 3 g (90 %) de cristaux blancs.

$C_{14}H_{19}NO_5$ : 281,3
F : 112°-117°
$[\alpha]_D$ : - 13,6° (c 0,3 % $CH_3$ OH)

4) Préparation de l'amino-3 didésoxy-3,4 β-D-xylo-hexopyrannoside de méthyle

A 3 g (0,0107 mole) du composé précédent dans du méthanol (45 ml) est ajoutée une solution molaire de méthylate de sodium (5 ml). Aprés deux heures d'agitation à température ambiante, le milieu réactionnel est évaporé sous pression réduite. Le résidu est purifié par chromatographie sur silice -éluant dichlorméthane-méthanol ammoniacal 17:3.

On isole 1,5 g du produit cristallisé.

$C_7H_{15}NO_4$ : 177,2
F : 155°-158° (acétonitrile)
$[\alpha]_D$ : - 43,5° (c 0,74 $CH_3OH$).

5) Préparation du [(chloro-2 éthyl)-3 nitroso-3 ureido]-3 didésoxy-3,4 β-D-xylo-hexopyrannoside de méthyle

$5.10^{-3}$ mole d'amino-3 didésoxy-3,4 β-D xylohexopyrannoside de méthyle sont mis en solution dans 2 ml de DMF anhydre, puis sont ajoutés, goutte à goutte, à 0° C et sous agitation $5.10^{-3}$ mole d'isocyanate de chloro-2 éthyle. Après 5 heures d'agitation, le mélange réactionnel est évaporé à sec sous vide. Le résidu est dissous dans 8 ml d'acide formique. A la solution maintenue à 0° C sont ajoutés par petites portions et sous agitation 0,036 mole de nitrite de sodium. Après 30 minutes, sont ajoutés 10 ml d'eau, l'agitation est alors maintenue 1 heure. Le mélange réactionnel est versé sur 100 ml d'acétate éthyle pur séché sur sulfate de sodium et évaporé à sec, puis purifié par cristallisation dans l'éther isopropylique.

Analyse :
$C_{10}H_{18}ClN_3O_6$ : 311,7 Rdt. : 49 %
F : 111°-112°
$[\alpha]_D$ : + 5,7° (c 1,4 % $CH_3OH$).

EXEMPLE 2 :

Préparation du [(chloro-2 éthyl)-3 nitroso-3 ureido]-3 benzoyl-6 didésoxy-3,4 β-D-xylo-hexopyrannoside de méthyle (IC. 1674)

On prépare de l'amino-3 benzoyl-6 didésoxy-3,4 β-D-xylo-hexopyrannoside de méthyle, comme il a été décrit à l'exemple 1, étape 3, puis l'amine obtenue est traitée comme décrit dans l'exemple 1, étape 5.

Analyse :
$C_{17}H_{22}ClN_3O_7$ : 415,83 Rdt. 60 %
F : 108°
$[\alpha]_D$ : + 7,3 (c 0,28 $CH_3OH$)

EXEMPLE 3 :

Préparation du chloro-4 [(chloro-2 éthyl)-3 nitroso-3 ureido]-3 didésoxy-3,4 β-D-galacto-hexopyrannoside de méthyle (IC. 1803)

1) Préparation de l'amino-3 benzoyl-6 chloro-4 didésoxy-3,4 β-D-galacto-hexopyrannoside de méthyle

A 3 g de l'azido-3 benzoyl-6 chloro-4 didésoxy-3,4 β-D-galacto-hexopyrannoside de méthyle dans 100 ml d'éthanol absolu et 2 ml de triéthylamine, on ajoute sous azote 500 mg de charbon palladié à 10 % et on agite sous atmosphère d'hydrogène pendant 24 heures.

Après élimination du catalyseur, le filtrat est évaporé ; le résidu est purifié par chromatographie sur silice.

Le produit est recristallisé dans l'éther éthylique.

Analyse :
$C_{14}H_{18}ClNO_5$ : 315,5 Rdt. : 82 %
F : 160°-162°
$[\alpha]_D$ = - 11,0° (c 0,85 $CHCl_3$)

2) Préparation de l'amino-3 chloro-4 didésoxy-3,4 β-D-galacto-hexopyrannoside de méthyle

On procède à la debenzoylation selon le procédé classique, à l'aide de méthylate de sodium.

Le produit obtenu a les caractéristiques suivantes :

Analyse :
$C_7H_{14}ClNO_4$ : 211,5
F : 158°-162°
$[\alpha]_D$ : - 4,0° (c 0,84 $CHCl_3$)

3) Préparation du chloro-4 [(chloro-2 éthyl)-3 nitroso-3 ureido]-3 didésoxy-3,4 β-D-galacto-hexopyrannoside de méthyle

On procède comme décrit à l'exemple 1, à l'étape 5, à partir du composé obtenu à l'issue de l'étape 2 ci-dessus.

Analyse :

$C_{10}H_{17}Cl_2N_3O_6$ : 346,17

F : 145°-148°

$[\alpha]_D$ : + 30° (c 0,65 MeOH).

EXEMPLE 4 :

Préparation du [(chloro-2 éthyl)-3 nitroso-3 ureido]-3 didésoxy-3,4 α-D-xylo-hexopyrannoside de méthyle (IC. 1677)

1) Préparation de l'azido-3 benzoyl-6 desoxy-3 α-D gluco-hexopyrannoside de méthyle

29 g d'azido-3 désoxy-3 D gluco-hexopyrannoside sont mis en solution dans 600 ml de méthanol chlorhydrique N puis portés au reflux 2 heures puis évaporés à sec sous vide.

Le produit est repris par du chlorure de méthylène puis lavé à l'eau jusqu'à absence d'ion chlorure. La phase organique séchée sur sulfate de sodium puis évaporée à sec sous vide donne un résidu de 31 g de produit glycosyle utilisé tel quel dans l'étape suivante.

Le produit est dissout dans 1 250 ml de toluène anhydre. On ajoute alors 90 ml d'oxyde de bis tributylétain et on porte 3 heures au reflux. On ramène le milieu réactionnel à - 15° et on additionne goutte à goutte 40 ml de chlorure de benzoyle dilué dans 200 ml de chlorure de méthylène. Après 24 heures d'agitation, le milieu réactionnel est évaporé à sec sous vide puis chromatographié sur silice (éluant $CH_2Cl_2CH_3OH$ 98:2).

On obtient ainsi sous forme de laque :

- 7,5 g d'azido-3 benzoyl-6 désoxy-3β-D glucohexopyrannoside de méthyle.
- 7,0 g d'azido-3 benzoyl-6 déoxy-3 α-D glucohexopyrannoside de méthyle.

2) Préparation de l'azido-3 benzoyl-6 chloro-4 didésoxy-3,4 α-D-galacto-hexopyrannoside de méthyle

A 4,8 g (0,015 mole) de l'azido-3 benzoyl-6 déoxy-3 α-D-gluco-hexopyrannoside de méthyle dans 250 ml de pyridine anhydre, on ajoute à 0°C et goutte à goutte, 12,5 ml (0,15 mole) de chlorure de sulfuryle. La solution est agitée pendant 18 heures à 0°C puis ramenée à température ambiante. Le milieu réactionnel est ensuite versé sur 500 g de glace, extrait par du dichlorométhane.

La phase organique, lavée à l'acide sulfurique N, puis à l'eau est séchée sur sulfate de sodium, filtrée et évaporée. Le résidu huileux, purifié par chromatographie sur silice éluant hexane-acétate d'éthyle 4:1, donne 4,05 g (80 %) de cristaux blancs.

$C_{14}H_{16}ClN_3O_5$ : 341,7

F : 104-106° (hexane-acétate d'éthyle)

$[\alpha]_D$ : + 140,5° (c 1,3 $CHCl_3$)

3) Préparation de l'amino-3 benzoyl-6 didésoxy-3,4 α-D-xylo-hexopyrannoside de méthyle

A 4,05 g (0,012 mole) du composé précédent dans 200 ml de toluène anhydre, est ajouté de l'azobisisobutyronitrile (700 mg, 4.27 mmoles), puis sous azote, goutte à goutte, de l'hydrure de tributylétain (12,6 ml, 47 mmoles). Le milieu réactionnel est porté au reflux pendant 2 heures puis évaporé sous pression réduite.

Par chromatographie sur silice éluant dichlorométhane-méthanol ammoniacal 19:1, on isole 3 g (90 %) de cristaux blancs.

4) Préparation de l'amino-3 didésoxy-3,4 α-D-xylo-hexopyrannoside de méthyle

A 3 g (0,0107 mole) du composé précédent dans du méthanol (45 ml) est ajoutée une solution molaire de méthylate de sodium (5 ml). Après deux heures d'agitation à température ambiante, le milieu réactionnel est évaporé sous pression réduite. Le résidu est purifié par chromatographie sur silice -éluant dichlorométhane-méthanol ammoniacal 17:3.

On isole 1,5 g du produit cristallisé.

F = 130-134°C

$[\alpha]_D$ : + 163° (c 0,9 $CHCl_3$)

5) Préparation du [(chloro-2 éthyl)-3 nitroso-3 ureido]-3 didésoxy-3,4 α-D-xylo-hexopyrannoside de méthyle

$5.10^{-3}$ mole d'amino-3 didésoxy-3,4 α-D xylohexopyrannoside de méthyle sont mis en solution dans 2 ml de DMF anhydre, puis sont ajoutés, goutte à goutte, à 0°C et sous agitation $5.10^{-3}$ mole d'isocyanate de chloro-2 éthyle. Après 5 heures d'agitation, le mélange réactionnel est évaporé à sec sous vide. Le résidu est dissous dans 8 ml d'acide formique. A la solution maintenue à 0°C sont ajoutés par petites portions et sous agitation 0,036 mole de nitrite de sodium. Après 30 minutes, sont ajoutés 10 ml d'eau, l'agitation est alors maintenue 1 heure. Le mélange réactionnel est versé sur 100 ml d'acétate éthyle pur séché sur sulfate de sodium et

évaporé à sec, puis purifié par cristallisation dans l'éther isopropylique.
Analyse : $C_{10}H_{18}ClN_3O_6$ : 311,7
F : 109°-110°
$[\alpha]_D$ : + 120,8° (c 0,48 % MeOH).

EXEMPLE 5 :

Préparation du chloro-4 [(chloro-2 éthyl)-3 nitroso-3 ureido]-3 didésoxy-3,4 α-D-galacto-hexopyrannoside de méthyle (IC. 1676)

1) Préparation de l'amino-3 benzoyl-6 chloro-4 didésoxy-3,4 α-D-galacto-hexopyrannoside de méthyle

A 3 g de l'azido-3 benzoyl-6 chloro-4 didésoxy-3,4 α-D-galacto-hexopyrannoside de méthyle, obtenu tel que décrit plus haut, dans 100 ml d'éthanol absolu et 2 ml de triéthylamine, on ajoute sous azote 500 mg de charbon palladié à 10 % et on agite sous atmosphère d'hydrogène pendant 24 heures.

Après élimination du catalyseur, le filtrat est évaporé ; le résidu est purifié par chromatographie sur silice.
Le produit est recristallisé dans l'éther éthylique.
Analyse :
$C_{14}H_{18}ClNO_5$ : 315,5
F : 161°-164°
$[\alpha]_D$ : + 118,5° (c 1,25 $CHCl_3$)

2) Préparation de l'amino-3 chloro-4 didésoxy-3,4 α-D-galacto-hexopyrannoside de méthyle

On procède à la debenzoylation selon le procédé classique, à l'aide de méthylate de sodium.
Le produit obtenu a les caractéristiques suivantes :
Analyse :
$C_7H_{14}ClNO_4$ : 211,5
F : 135-138°
$[\alpha]_D$ : + 184° (c 0,92 MeOH)

3) Préparation du chloro-4 [(chloro-2 éthyl)-3 nitroso-3 ureido]-3 didésoxy-3,4 α-D-galacto-hexopyrannoside de méthyle

On procède comme décrit à l'exemple 1, à l'étape 5, à partir du composé obtenu à l'issue de l'étape 2 ci-dessus.
Analyse :
$C_{10}H_{17}Cl_2N_3O_6$ : 346,17
F : 129°-130°
$[\alpha]_D$ : + 139,3° (c 0,5 $CH_3OH$).

ETUDE PHARMACOLOGIQUE :

Afin de tester l'activité antitumorale des composés de l'invention décrits plus haut, on a utilisé en premier lieu la leucémie murine L1210.

On a également utilisé comme tumeur le mélanome B16.

En particulier, on a testé les produits de l'invention vis-à-vis du mélanome B16, en les administrant par voie I.P. et par voie I.V.

On a également testé les produits de l'invention vis-à-vis du carcinome C38 du colon en les administrant par voie I.P. et par voie I.V.

En pratique, les effets biologiques des nouveaux dérivés de nitrosourées conformes à la présente invention ont été testés comme suit :

PROTOCOLE DE TRAITEMENT DES PRODUITS DE L'INVENTION

I - MATERIEL ET METHODES

A - Tumeurs utilisées

Deux tumeurs murines ont été utilisées pour les études "in vivo" : la leucémie L1210 et le mélanome B16.

1) LEUCEMIE L1210

. Animaux

Les expérimentations ont toujours été effectuées sur des souris femelles Indemnes d'Organismes Pathogènes Spécifiques (I.O.P.S.),
- soit de lignée DBA/2Jlco
- soit B6 D2 F1/Jlco (hybrides de première génération entre les lignées C57BL/6 et DBA/2).

éliminer les fragments mal dilacérés, la suspension cellulaire homogène obtenue est numérée à l'aide d'une cellule de MALASSEZ, et ramenée à la concentration voulue ($4x10^6$ cellules tumorales par ml) par dilution dans du milieu NCTC 109.

. Répartition en séries expérimentales

Cette répartition s'effectue comme décrit précédemment pour la leucémie L1210.

B- Protocole de traitement

Les doses des produits de l'invention, utilisées lors des différentes expérimentations sont exprimées en milligrammes par kilogramme de poids corporel.

Les produits sont mis en solution dans le soluté injectable de chlorure de sodium isotonique.

Deux protocoles de traitement ont été utilisés lors des différentes expérimentations :

- soit une injection unique à J1, par voie intrapéritonéale (I.P.), ou intraveineuse (I.V.),
- soit trois injections I.P. à J1, J5, J9.

Dans chaque expérimentation, les animaux de la série témoin reçoivent suivant le protocole expérimental, une ou plusieurs injections, par la même voie (I.P. ou I.V.) d'un même volume (0,2 ml/20 g) du véhicule sans principe actif (soluté isotonique de chlorure de sodium).

II- EXPRESSION DES RESULTATS

Pour chaque expérimentation un tableau précise :

- le nombre de souris survivantes à J60 avec le pourcentage correspondant,
- pour les séries traitées le T/C x 100

. T représentant le temps médian de survie des souris de la série traitée,

. C représentant le temps médian de survie des souris de la série contrôle (témoin).

III- REMARQUE

Les lignées de souris utilisées, les protocoles expérimentaux et le mode d'expression des résultats sont en conformité avec l'instruction 271 F de la "N.C.I. Division of Cancer Treatment" (novembre 1983).

IV- RESULTATS PHARMACOLOGIQUES :

1°) Etude toxicologique :

Les résultats relatifs à la toxicologie sont rassemblés dans le tableau I ci-après :

TABLEAU I

| IC | $DL_0$ mg/kg | $DL_{50}$ mg/kg | $DL_{90}$ mg/kg |
|---|---|---|---|
| 1674 | ≥40 | - | - |
| 1675 | ≥20 | sensiblement égal à 40 | - |
| 1676 | ≥20 | - | - |
| 1677 | - | - | ≤20 |

2°) Activité des produits IC. 1674, 1675, 1677 par voie i.p. sur la leucémie L1210 souche USA et le mélanome B16

Composé de l'invention IC. 1674 :

L1210 souche USA - $B_6D_2F_1$ - Traitement I.P. à J1, J5, J9
3 x 1,25 mg/kg, 3 x 5 mg/kg

| | T/C | Nombre de survivants à J60 |
|---|---|---|
| 3x1,25 | 111 | 0/10 |
| 3 x 5 | 130 | 0/10 |

Composé de l'invention IC. 1675 :

L1210 souche USA - $B_6D_2F_1$ - Traitement I.P. à J1, J5, J9
3 x 1,25 mg/kg, 3 x 5 mg/kg

| | T/C | Nombre de survivants à J60 |
|---|---|---|
| 3x1,25 | 164 | 0/10 |
| 3 x 5 | >600 | 5/10 |

Mélanome B16 - $B_6D_2F_1$ - Traitement à J1, J5, J9
3 x 10 mg/kg

| T/C | Nombre de survivants à J60 |
|---|---|
| 258 | 8/10 |

Composé de l'invention IC. 1676 :

L1210 souche USA - $B_6D_2F_1$ - Traitement I.P. à J1, J5, J9
3 x 125 mg/kg, 3 x 5 mg/kg

| | T/C | Nombre de survivants à J60 |
|---|---|---|
| 3x1,25 | 136 | 0/10 |
| 3 x 5 | 188 | 0/10 |

Mélanome B16 - $B_6D_2F_1$ - Traitement I.P. à J1, J5, J9
3 x 10 mg/kg

| T/C | Nombre de survivants à J60 |
|---|---|
| 202 | 3/10 |

Composé de l'invention IC. 1677 :

L1210 souche USA - $B_6D_2F_1$ - Traitement I.P. à J1, J5, J9
3 x 1,25 mg/kg, 3 x 5 mg/kg

| | T/C | Nombre de survivants à J60 |
|---|---|---|
| 3x1,25 | 157 | 0/10 |
| 3 x 5 | >600 | 6/10 |

Mélanome B16 - $B_6D_2F_1$ - Traitement I.P. à J1, J5, J9
3 x 10 mg/kg

| T/C | Nombre de survivants à J60 |
|---|---|
| 257 | 7/10 |

3°) Activité des composés de l'invention après administration par voie intra veineuse sur le mélanome B16 :

L'activité des composés de l'invention a également été testée, en administrant lesdits composés de l'invention par voie I.V.

Des souris B6C3F1 femelles reçoivent, par voie S.C. (sous cutanée), 0,5 ml d'un homogénat de tumeur (1g/10 ml) au jour 0. Les produits de l'invention et les composés de comparaison sont administrés aux jours 3, 7 et 11 en solution isotonique.

On donne ci-après, dans le tableau II, les résultats concernant l'action du composé IC. 1675 administré par voie I.V. sur la croissance tumorale du mélanome B16 implantée par voie sous cutanée, en comparaison avec la carmustine BCNU commercialisée sous le nom de BICNU®.

Dans le tableau II ci-dessous et dans les tableaux ci-après,
T représente le poids moyen (ou médian) des tumeurs chez les souris traitées,
C représente le poids moyen (ou médian) des tumeurs chez les souris témoins non traitées et le rapport T/C % correspond à T/C x 100.

TABLEAU II

| Produit | Dose mg/kg/inj. | Poids Médian de la tumeur au jour 3(mg) | Poids Médian de la tumeur au jour 30(mg) | T/C au jour 30 % |
|---|---|---|---|---|
| IC1675 | 20 | 36,0 | 688 | 8,8 |
| | 15 | 23,0 | 1 044 | 13,4 |
| | 10 | 32,0 | 2 025 | 25,9 |
| BCNU | 20 | 40 | 2 581 | 33,0 |
| TEMOINS | 0 | 40 | 7 812 | 100 |

Dans ce cas, il faut noter que le chiffre est d'autant plus petit que l'activité est meilleure, dans le cas idéal, le poids de la tumeur chez la souris traitée est de 0 mg, et donc le rapport T/C % est égal à 0.

Les résultats obtenus montrent que les composé de l'invention sont actifs lorsqu'ils sont administrés par voie I.V., ce qui les rend tout à fait appropriés à une utilisation en thérapeutique humaine.

4°) Activité du composé de l'invention IC 1675 administré par voie I.P. à des souris porteuses du carcinome C38 du colon, implanté par voie S.C.

Un fragment de tumeur est implanté au jour 0 chez des souris $B_6D_2F1$ femelles (19-22g). Le traitement est administré par voie I.P., pour tous les produits, aux jours 2 et 9, ce qui constitue un traitement retardé. Le poids des tumeurs est calculé au jour 20.

Les résultats figurent dans le tableau III ci-après.

TABLEAU III

| Produit | Dose mg/kg/inj. | Poids Médian de la tumeur au jour 20 (mg) | T/C % | Nombre de souris sans tumeur/total + remarques |
|---|---|---|---|---|
| IC 1675 | 30 | 0 | 0 | 7/7 et 5 souris mortes |
| | 20 | 0 | 0 | 7/9 |
| | 10 | 272 | 67 | 1/10 |
| TCNU | 30 | - | - | 10 morts précoces |
| | 15 | 0 | 0 | 7/10 |
| | 7,5 | 266 | 65 | 2/10 |
| TEMOINS | 0 | 405 | 100 | 0/32 |

Le tableau III ci-dessus fait apparaître l'efficacité importante du composé de l'invention IC 1675 quand il est administré par voie I.P., en traitement retardé vis-à-vis du carcinome C38 du colon. Cette activité est prédictive d'une activité chez l'homme pour le traitement de diverses tumeurs.

5°) Activité antitumorale du composé IC 1675 sur le carcinome C38 du colon : comparaison des voies intraveineuses et intrapéritonéales

Dans le but de vérifier l'activité antitumorale du produit de l'invention IC 1675 quand il est administré par voie I.V., une autre expérience a été réalisée dans laquelle ce produit est administré par voie I.P. et voie I.V. comparativement.

Cette expérience est réalisée dans des conditions particulièrement difficiles, le premier jour du traitement correspondant au 8e jour après l'implantation du carcinome C38, tandis que de façon classique, le traitement débute le jour d'implantation de la tumeur (J0) ou 1 jour après (J1) l'implantation de la tumeur.

Le poids médian des tumeurs au premier jour de traitement (jour 8) est de 12 mg (tumeurs de 8 à 40 mg). Les animaux sont traités les jours 8, 12 et 16 selon les différentes voies indiquées.

Les résultats relatifs à l'action du composé de l'invention IC 1675 sur des souris porteuses de l'adénocarcinome 38 du colon sont rassemblés dans le tableau IV ci-après

TABLEAU IV

| Produit | Dose mg/kg/inj. | Voie I.P. Poids médian de la tumeur J20 | Voie I.P. Poids médian de la tumeur J27 | Voie I.P. T/C% J20 | Voie I.P. T/C% J27 | Voie I.P. souris sans tumeur/total J20 | Voie I.P. souris sans tumeur/total J27 | Voie I.V. Poids médian de la tumeur J20 | Voie I.V. Poids médian de la tumeur J27 | Voie I.V. T/C% J20 | Voie I.V. T/C% J27 | Voie I.V. souris sans tumeur/total J20 | Voie I.V. souris sans tumeur/total J27 |
|---|---|---|---|---|---|---|---|---|---|---|---|---|---|
| IC 1675 | 20 | 13 | 172 | 2 | 13 | 1/8 | 2/8 | 0 | 0 | 0 | 0 | 6/8 | 6/6 |
| | 15 | 88 | 726 | 15 | 56 | 0 | 0 | 0 | 0 | 0 | 0 | 7/8 | 7/7 |
| | 10 | 324 | 1099 | 57 | 84 | 0 | 0 | 23 | 352 | 4 | 27 | 0/8 | 0/8 |
| BCNU | 20 | 196 | 274 | 34 | 21 | 0/8 | 0/6 | | | | | | |
| TEMOINS | 0 | 586 | 1295 | 100 | | 0/24 | | 586 | 1295 | 100 | | 0/24 | |

On constate que non seulement l'activité des composés de l'invention vis-à-vis du carcinome C38 est remarquable, et qu'elle est encore supérieure après une administration par voie I.V. par rapport à l'administration I.P., puisqu'à des doses ne produisant aucune toxicité (15 mg/kg/inj.), on peut observer une régression tumorale totale . En effet, on voit disparaître au 20e jour une tumeur mesurable au début du traitement, et il n'y a pas récidive au 27e jour. Ceci est démontré par le nombre de souris sans tumeur au 27e jour.

CONCLUSION GENERALE :

L'expérimentation animale effectuée avec les produits selon l'invention donne de très intéressants résultats dans les modèles utilisés : tumeur L1210 USA et mélanome B16, et d'excellents résultats dans le modèle du carcinome du colon C38.

L'ensemble des résultats obtenus sur ces modèles montre que les composés de l'invention ont un spectre d'action plus large que les autres composés antitumoraux connus à ce jour.

Les composés de l'invention présentent en outre le remarquable avantage de permettre la régression de tumeurs animales très difficiles à combattre avec les antitumoraux connus à ce jour.

Les composés de formule I ci-dessus sont donc des substances thérapeutiquement actives et, sous cet aspect, ils représentent un autre objet de la présente invention.

Les composés selon l'invention sont particulièrement appropriés pour le traitement de divers cancers humains, notamment sont sensibles à la chimiothérapie. Ils sont particulièrement appropriés au traitement des tumeurs broncho-pulmonaires, des tumeurs de la sphère ORL, des tumeurs digestives (gastrique, pancréatique, colique et rectale), des tumeurs du sein, des tumeurs génitales, des tumeurs osseuses (ostéosarcomes, réticulo-sarcomes), des mélanomes, des hémato-sarcomes (lymphomes hodgkiniens et non hodgkiniens), des myélomes multiples.

L'invention concerne aussi les compositions pharmaceutiques comprenant les composés nouveaux susdits en association avec un véhicule pharmaceutique approprié au mode d'administration choisi.

L'invention concerne particulièrement des solutions stériles injectables propres à être administrées par injections ou perfusions intraveineuses. Elle concerne, en particulier, des solutions hydroalcooliques physiologiquement acceptables.

Les produits selon l'invention, peuvent par exemple être présentés sous forme de poudre lyophilisée, laquelle, pour être administrée, est préparée extemporanément par solubilisation à l'aide d'un solvant alcoolique stérile. La solution ainsi obtenue est ensuite diluée avec de l'eau stérile apyrogène, puis avant d'être administrée par perfusion intraveineuse, la solution est à nouveau rediluée dans du soluté isotonique chloruré ou glucosé.

Les doses administrées doivent être suffisantes pour qu'une action puisse se manifester au moins dans une

proportion relativement importante de patients atteints de l'une ou l'autre des diverses formes de cancer qui sont ou seront accessibles à la chimiothérapie, cependant sans pour autant excéder celles pour lesquelles les composés deviendraient trop toxiques.

A titre d'exemple, les doses administrées par voie générale, notamment par perfusion, et exprimées en mg/kg pourront varier d'environ 0,1 à environ 5 mg/kg, par exemple d'environ 1 mg/kg.

L'invention concerne également les autres formes d'administration, notamment, par voie orale (compositions solides ou liquides).

Ces intervalles de doses n'ont évidemment qu'une valeur d'indication.

Il est naturellement entendu que, dans ce type de thérapie, les doses administrées doivent, dans chaque cas, être évaluées par le clinicien, compte tenu de l'état du malade et de sa réactivité personnelle à l'égard des médicaments.

Un exemple de préparation pharmaceutique des produits selon l'invention comprend de 10 à 250 mg, notamment 50 mg, de l'un au moins des produits selon l'invention, présenté sous forme de poudre lyophilisée stérile, en association avec une ampoule de solvant physiologiquement acceptable, notamment du sérum physiologique, à raison de 5 ml par ampoule.

Du fait de leur activité particulièrement importante, les composés de l'invention sont également utiles comme produits de référence dans des études pharmacologiques, notamment, aux fins de comparaison de propriétés antitumorales de produits étudiés par rapport à un produit de référence.

**Revendications**

1. Dérivés de nitrosourées de formule I

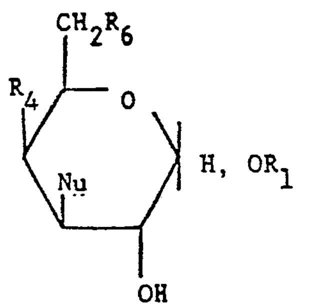

I

sous forme de l'un des deux anomères α ou β,

dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente H ou Hal, Hal étant un atome d'halogène, notamment Cl,
- $R_6$ représente OH ou $O-\underset{\underset{O}{\|}}{C}-R$,

  R représentant un groupe alcoyle de 1 à 6 atomes de carbone, un groupe aryle non substitué ou substitué sur le noyau aromatique par un ou plusieurs atomes, notamment jusqu'à 3 atomes d'halogène, groupes $NO_2$, $CF_3$ ou groupes alcoxy de 1 à 4 atomes de carbone,

-Nu représente le groupe $NH-\underset{\underset{O}{\|}}{C}-\underset{\underset{NO}{|}}{N}-CH_2-CH_2-Hal'$, Hal' représentant un halogène identique ou différent de Hal et notamment le chlore.

2. Dérivés de nitrosourées selon la revendication 1, de formule I,

dans laquelle

-$R_1$ représente un groupe alcoyle de 1 à 12 atomes de carbone,
- $R_4$ représente un atome d'halogène,
- $R_6$ représente les significations indiquées à la revendication 1.

3. Dérivés de nitrosourées selon la revendication 1, de formule I,

dans laquelle

- $R_1$ représente un groupe $CH_3$,
- $R_4$ représente un atome d'halogène,
- $R_6$ représente les significations indiquées à la revendication 1.

4. Dérivés de nitrosourées selon la revendication 1, de formule I, dans laquelle
- $R_1$ représente un groupe $CH_3$,
- $R_4$ représente Cl,
- $R_6$ représente les significations indiquées à la revendication 1.

5. Dérivés de nitrosourées selon la revendication 1, de formule I, dans laquelle
- $R_1$ représente un groupe $CH_3$,
- $R_4$ représente un halogène,
- $R_6$ représente $O\underset{\underset{O}{\|}}{C}R$, R ayant la signification indiquée à la revendication 1.

6. Dérivés de nitrosourées, selon la revendication 1, de formule I, dans laquelle
- $R_1$ représente un groupe $CH_3$,
- $R_4$ représente l'hydrogène,
- $R_6$ représente OH.

7. Dérivés de nitrosourées, selon la revendication 1, de formule I, dans laquelle
- $R_1$ représente un groupe $CH_3$,
- $R_4$ représente l'hydrogène,
- $R_6$ représente $O-\underset{\underset{O}{\|}}{C}-R$, R ayant la signification indiquée à la revendication 1.

8. Dérivé de nitrosourées choisi parmi ceux ayant des formules suivantes

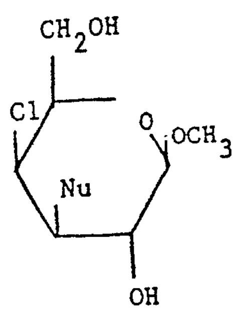

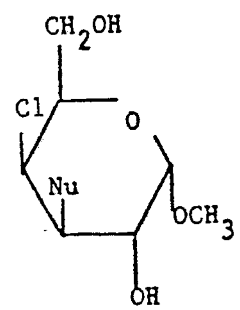

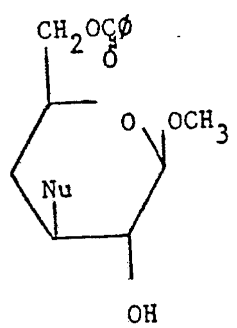

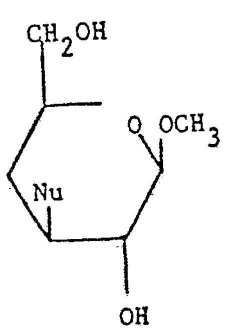

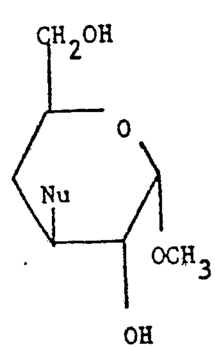

9. Procédé de préparation de dérivés de nitrosourées de formule I

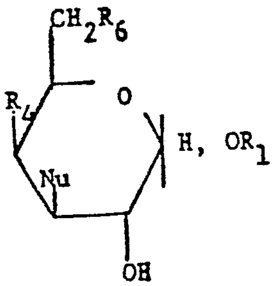

I

sous forme de l'un des deux anomères α ou β, dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente Hal, Hal étant un atome d'halogène, notamment Cl,
- $R_6$ représente OH ou $\text{O-}\underset{\underset{\displaystyle O}{\|}}{C}\text{-R}$,

  R représentant un groupe alcoyle de 1 à 6 atomes de carbone, un groupe aryle non substitué ou substitué sur le noyau aromatique par un ou plusieurs atomes, notamment jusqu'à 3 atomes d'halogène, par un à trois grouped $NO_2$, $CF_3$ ou groupes alcoxy de 1 à 4 atomes de carbone,
- Nu représente le groupe $\text{NH-}\underset{\underset{\displaystyle O}{\|}}{C}\text{-}\underset{\underset{\displaystyle NO}{|}}{N}\text{-}\ CH_2\text{-}CH_2\text{-Hal}'$, Hal' identique à Hal ou différent de Hal et représentant un halogène, notamment le chlore, caractérisé en ce que

a) dans une première série de réactions, on fait réagir le composé de départ de formule II

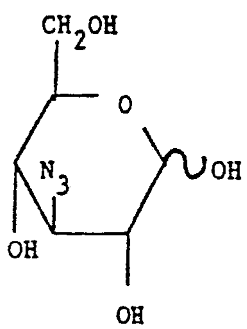

II

sous forme de mélange des anomères α et β avec un agent de glycosylation, constitué par un alcool $R_1OH$, $R_1$ ayant la signification indiquée ci-dessus, pour transformer le groupe OH en position 1, en groupe $OR_1$,

* on traite le produit ainsi obtenu avec un dérivé fonctionnel de l'acide R-COOH pour transformer le groupe OH en position 6, en groupe $\text{O-}\underset{\underset{\displaystyle O}{\|}}{C}\text{-R}$,

* on sépare les anomères α et β par une méthode classique,

* on fait réagir chacun des anomères α ou β avec un agent d'halogénation pour introduire un halogène en position 4,

le composé obtenu étant sous forme de l'un des anomères α ou β et répondant à la formule III

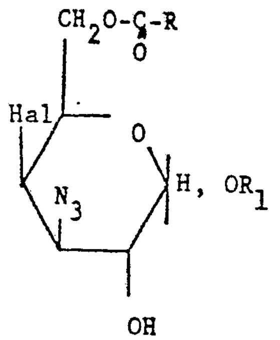

III

dans laquelle

- R et $R_1$ ont les significations indiquées dans la formule I,
- Hal représente un halogène,

b) ensuite, dans une deuxième étape, on effectue la réduction du composé de formule III, pour réduire $N_3$ en $NH_2$, cette réduction étant en outre effectuée dans des conditions telles que l'halogène en position 4 soit ou non réduit en hydrogène, cette réduction étant éventuellement suivie de l'élimination du groupe $O-\underset{\underset{O}{\|}}{C}-R$ en position 6 et son remplacement par un groupe OH,
le composé obtenu à l'issue de cette deuxième étape étant sous forme de l'un des anomères α ou β, et répondant à la formule IV suivante :

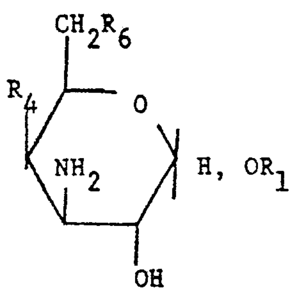

IV

dans laquelle
$R_1$, $R_4$, $R_6$ ont les significations indiquées dans la formule I,
c) enfin, dans une troisième série de réactions, on effectue la réaction des anomères α ou β de formule IV sur un isocyanate d'halogéno éthyle pour transformer $NH_2$ en $NH\underset{\underset{O}{\|}}{C}NHCH_2CH_2Hal'$ et on traite le produit ainsi obtenu par un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$, afin d'obtenir le composé de formule I, sous forme de l'un des anomères α ou β.

10. Procédé de préparation selon la revendication 9, de dérivés de nitrosourées de formule I caractérisé en ce qu'il comprend

- la réaction du composé de formule II sous forme de mélange des anomères α et β

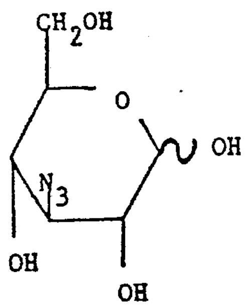

II

avec un agent de glycosylation constitué par $R_1OH$, $R_1$ ayant la signification indiquée à la revendication 9, pour donner le composé de formule V

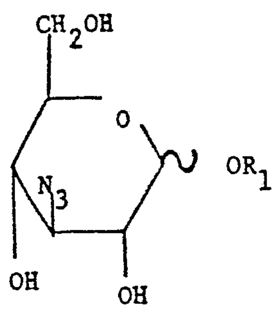

V

- l'introduction d'un groupe acyloxy en position 6, de formule $O-\underset{\underset{O}{\|}}{C}-R$, R ayant la signification indiquée à la revendication 9, pour donner le composé de formule

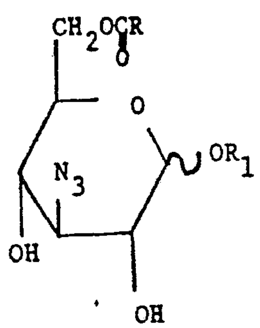

VI

- la séparation du composé VI en deux anomères α et β,

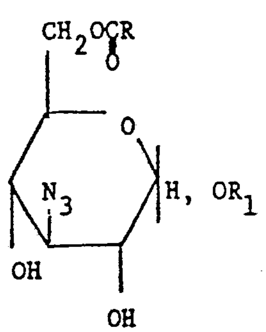

VII

- la réaction des anomères α ou β, de formule VII, avec un agent d'halogénation, pour donner les

anomères α ou β de formule III

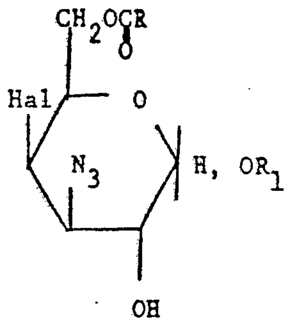

III

. puis

* soit la réduction des anomères α ou β de formule III ainsi obtenus, dans des conditions telles que $N_3$ soit réduit en $NH_2$, sans hydrogénolyse de l'halogène en position 4, pour donner les anomères α ou β de formule IX

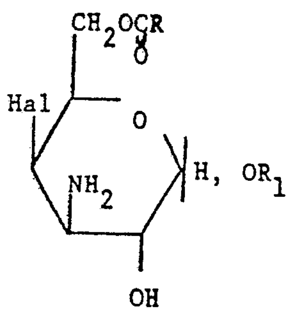

IX

cette réduction étant

** ou bien suivie de la réaction des anomères α ou β de formule IX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$, afin d'obtenir les composés de formule I, dans lesquels $R_4$ représente un halogène et $R_6$ représente $O-\underset{\underset{O}{\|}}{C}-R$,

** ou bien suivie de l'élimination à partir des anomères α ou β de formule IX du groupe $O-\underset{\underset{O}{\|}}{C}-R$, en position 6, et le remplacement par un groupe OH, puis la réaction des anomères α ou β de formule IV sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$, pour donner les composés de formule I, dans lesquels

$R_4$ représente un halogène et

$R_6$ représente OH,

. ou

*soit la réduction respectivement des anomères III dans des conditions telles que l'halogène en position 4 soit remplacé par H et le groupe $N_3$ soit réduit en $NH_2$, pour donner les anomères α ou β de formule X

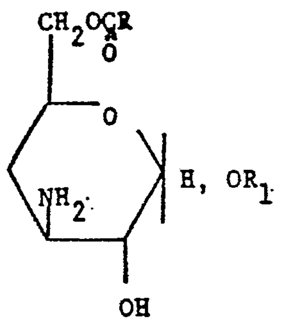

X

cette réduction étant suivie

** ou bien de la réaction des anomères α ou β de formule X sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\cdot\ H_2CH_2Hal'$,

respectivement pour obtenir les composés de formule I dans lesquels

$R_4$ représente H,

$R_6$ représente $O\text{-}\underset{\underset{O}{\|}}{C}\text{-}R$

** ou bien de l'élimination à partir des anomères α ou β de formule X du groupe $O\text{-}\underset{\underset{O}{\|}}{C}\text{-}R$ en position 6 et

son remplacement par un groupe OH pour donner les anomères α ou β de formule XI

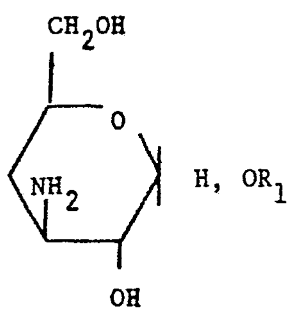

XI

suivi de la réaction des anomères α ou β de formule XI sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\cdot\ H_2CH_2Hal'$,

pour obtenir des anomères α ou β de formule I dans lesquels $R_4$ représente H, et $R_6$ représente OH.

11. Procédé de préparation selon les revendications 9 et 10 de dérivés de nitrosourées de formule I, catactérisé en ce que la réduction des composés de formule III, telle que $N_3$ soit réduit en $NH_2$ et Hal soit remplacé par l'hydrogène, est effectuée à l'aide d'hydrure de tributylétain, en présence d'azo 2-2' bis isobutyronitrile.

12. Procédé de préparation selon les revendications 9 et 10 de dérivés de nitrosourées de formule I, caractérisé en ce que le composé de formule II est soumis, après la glycosylation, à la réaction de l'oxyde de bis tribulétain et du chlorure de benzoyle, pour introduire le groupe benzoyle en position 6 et obtenir le composé de formule III, dans lequel R représente le groupe phényl sous forme de mélanges d'anomères α et β, ceux-ci pouvant être ensuite séparés.

13. Procédé selon la revendication 9 de préparation de dérivés de nitrosourées de formule I

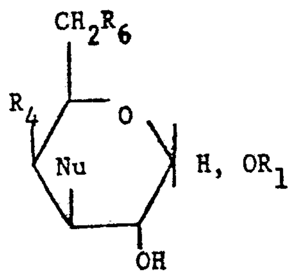

dans laquelle $R_1$, $R_4$, $R_6$ et Nu ont les significations indiquées à la revendication 9, caractérisé en ce qu'il comprend

- la glycosylation du composé de formule II sous forme du mélange d'anomères α et β

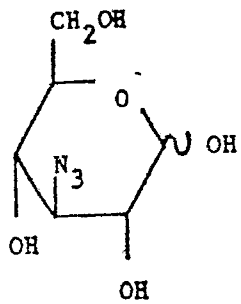

II

à l'aide de $CH_3OH$, pour obtenir le composé de formule XII

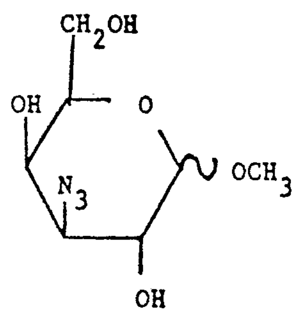

XII

- la réaction du composé de formule XII précédemment obtenu avec du chlorure de benzoyle et de l'oxyde de tributylétain pour obtenir le composé de formule

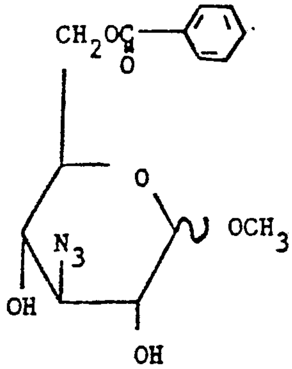

XIII

- la séparation des deux anomères α et β à partir du composé de formule XIII
- la réaction de l'un ou l'autre des anomères α ou β avec $SO_2Cl_2$, pour obtenir les anomères α ou β de formule

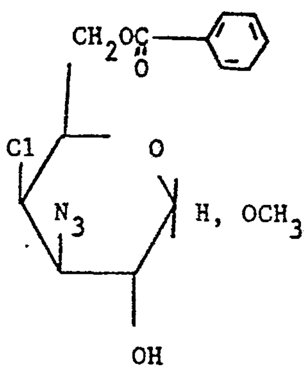

XIV

et

. soit l'hydrogénation catalytique des anomères α ou β de formule XIV précédemment obtenus pour donner les anomères α ou β de formule XV

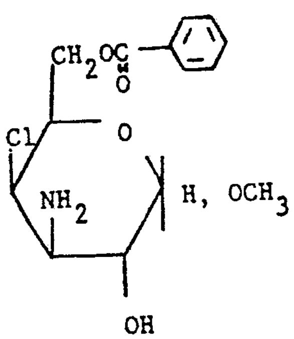

XV

suivi

.. soit de la réaction des anomères α ou β de formule XV sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

et pour donner les anomères α ou β de formule

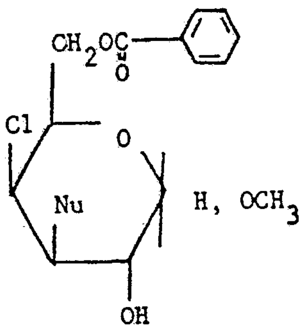

XVI

.. soit de la réaction sur les anomères α ou β de formule XV sur une base, notamment un alcoolate alcalin, pour éliminer le groupe benzoyle et donner les anomères α ou β de formule XVII

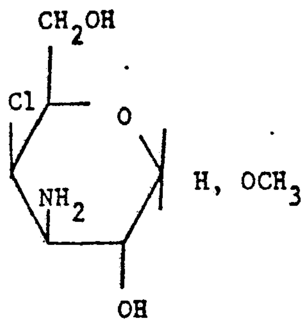

XVII

. suivi de la réaction des anomères α ou β de formule XVII sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\ \underset{\underset{O}{\|}}{C}\underset{\underset{NO}{|}}{N}\ H_2CH_2Hal'$,
et pour donner les anomères α ou β de formule XVIII

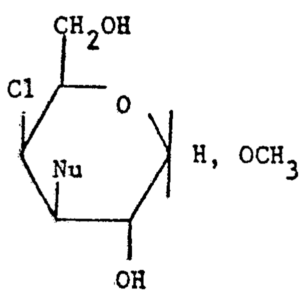

XVIII

. soit la réduction des anomères α ou β de formule XIV, à l'aide d'hydrure de tributylétain, en présence d'azo 2-2' bis isobutyronitrile, pour donner les anomères α ou β de formule XIX

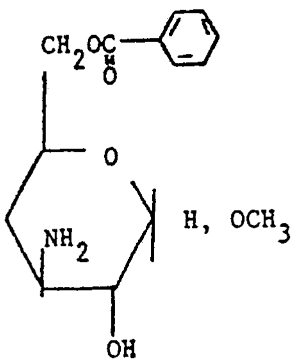

XIX

suivi

.. soit de la réaction des anomères α ou β de formule XIX sur un isocyanate d'halogéno éthyle pour transformer $NH_2$ en $NH\underset{O}{\underset{\|}{C}}NHCH_2CH_2Hal'$ et de l'action d'un nitrite de métal alcalin, notamment le nitrite de sodium, pour transformer $NH\underset{O}{\underset{\|}{C}}NHCH_2CH_2Hal'$ en $NH\underset{O}{\underset{\|}{C}}\underset{NO}{\underset{|}{N}}\ H_2CH_2Hal'$, et pour donner les anomères α ou β de formule XX

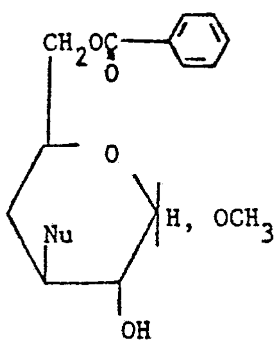

XX

.. soit de la réaction des anomères α ou β de formule XIX avec une base, notamment un alcoolate alcalin, pour éliminer le groupe benzoyle et donner les anomères α ou β de formule XXI

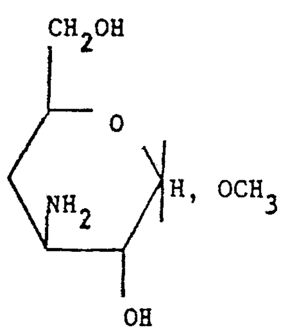

XXI

suivi de la réaction des anomères α ou β de formule XXI sur un isocyanate d'halogéno éthyle pour transformer $NH_2$ en $NH\underset{O}{\underset{\|}{C}}NHCH_2CH_2Hal'$ et de l'action d'un nitrite de métal alcalin, notamment le nitrite de sodium, pour transformer $NH\underset{O}{\underset{\|}{C}}NHCH_2CH_2Hal'$ en $NH\underset{O}{\underset{\|}{C}}\underset{NO}{\underset{|}{N}}\ H_2CH_2Hal'$, et pour donner les anomères α ou β de formule XXII

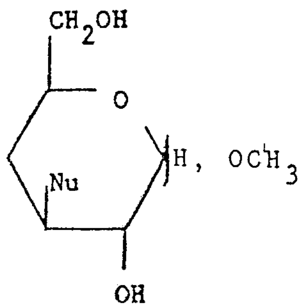

XXII

14. Composés de formule III

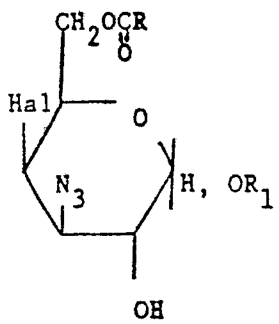

III

dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- R représente un groupe alcoyle de 1 à 6 atomes de carbone, un groupe aryle non substitué ou substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- Hal représente un halogène, notamment le chlore, sous forme de l'un de ses anomères $\alpha$ ou $\beta$.

15. Composés selon la revendication 14 de formule III dans laquelle

- $R_1$ représente un groupe alcoyle de 1 à 12 atomes de carbone,
- R représente

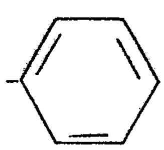

16. Composé de formule

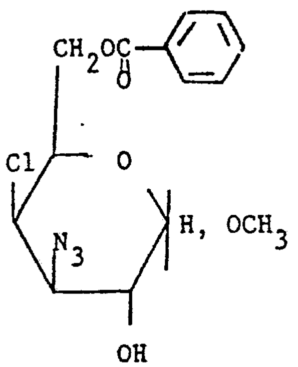

sous forme de l'anomère α ou β.

17. Les composés selon l'une quelconque des revendications 1 à 8 en tant que substance thérapeutiquement active.

18. Composition pharmaceutique caractérisée en ce qu'elle comprend l'un quelconque des composés selon les revendications 1 à 8, en association avec un véhicule pharmaceutiquement acceptable.

19. Composition pharmaceutique selon la revendication 18, caractérisée en ce qu'elle comprend 10 à 250 mg, notamment 50 mg, de l'un au moins des composés selon l'une quelconque des revendications 1 à 8, présenté sous forme de poudre lyophylisée stérile, en association avec une ampoule d'un solvant physiologiquement acceptable, notamment du sérum physiologique, à raison de 5 ml par ampoule.

**Revendications pour l'Etat contractant suivant : GR**

1. Dérivés de nitrosourées de formule I

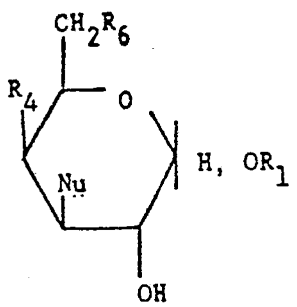

I

sous forme de l'un des deux anomères α ou β,
dans laquelle
- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente H ou Hal, Hal étant un atome d'halogène, notamment Cl,
- $R_6$ représente OH ou $O-\underset{\underset{O}{\|}}{C}-R$,

R représentant un groupe alcoyle de 1 à 6 atomes de carbone, un groupe aryle non substitué ou substitué sur le noyau aromatique par un ou plusieurs atomes, notamment jusqu'à 3 atomes d'halogène, groupes $NO_2$, $CF_3$ ou groupes alcoxy de 1 à 4 atomes de carbone,

- Nu représente le groupe $NH-\underset{\underset{O}{\|}}{C}-\underset{\underset{NO}{|}}{N}-CH_2-CH_2-Hal'$, Hal' représentant un halogène identique ou différent de Hal et notamment le chlore.

2. Dérivés de nitrosourées selon la revendication 1, de formule I,
dans laquelle
- $R_1$ représente un groupe alcoyle de 1 à 12 atomes de carbone,
- $R_4$ représente un atome d'halogène,
- $R_6$ représente les significations indiquées à la revendication 1.

3. Dérivés de nitrosourées selon la revendication 1, de formule I,
dans laquelle
- $R_1$ représente un groupe $CH_3$,

- $R_4$ représente un atome d'halogène,
- $R_6$ représente les significations indiquées à la revendication 1.

4. Dérivés de nitrosourées selon la revendication 1, de formule I, dans laquelle
- $R_1$ représente un groupe $CH_3$,
- $R_4$ représente Cl,
- $R_6$ représente les significations indiquées à la revendication 1.

5. Dérivés de nitrosourées selon la revendication 1, de formule I, dans laquelle
- $R_1$ représente un groupe $CH_3$,
- $R_4$ représente un halogène,
- $R_6$ représente $O\underset{\underset{O}{\|}}{C}R$, R ayant la signification indiquée à la revendication 1.

6. Dérivés de nitrosourées, selon la revendication 1, de formule I, dans laquelle
- $R_1$ représente un groupe $CH_3$,
- $R_4$ représente l'hydrogène,
- $R_6$ représente OH.

7. Dérivés de nitrosourées, selon la revendication 1, de formule I, dans laquelle
- $R_1$ représente un groupe $CH_3$,
- $R_4$ représente l'hydrogène,
- $R_6$ représente $O-\underset{\underset{O}{\|}}{C}-R$, R ayant la signification indiquée à la revendication 1.

8. Dérivé de nitrosourées choisi parmi ceux ayant des formules suivantes

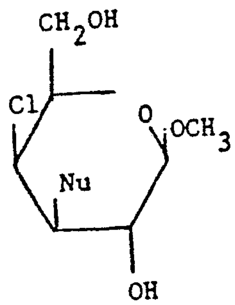

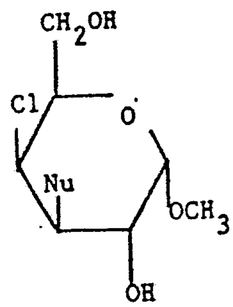

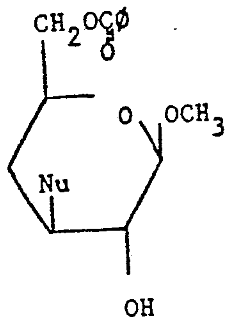

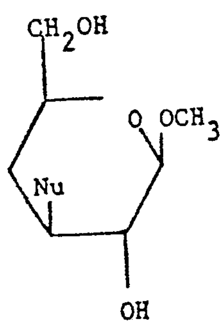

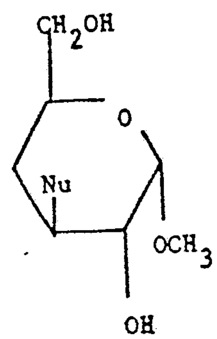

9. Procédé de préparation de dérivés de nitrosourées de formule I

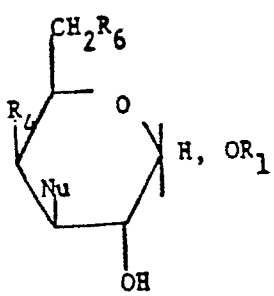

I

sous forme de l'un des deux anomères α ou β,
dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente Hal, Hal étant un atome d'halogène, notamment Cl,
- $R_6$ représente OH ou $O-\underset{\underset{O}{\|}}{C}-R$,

R représentant un groupe alcoyle de 1 à 6 atomes de carbone, un groupe aryle non substitué ou substitué sur le noyau aromatique par un ou plusieurs atomes, notamment jusqu'à 3 atomes d'halogène, par un à trois grouped $NO_2$, $CF_3$ ou groupes alcoxy de 1 à 4 atomes de carbone,

- Nu représente le groupe NH- $\underset{\underset{O}{\|}}{C}-\underset{\underset{NO}{|}}{N}-$ $CH_2$-$CH_2$-Hal', Hal' identique à Hal ou différent de Hal et représentant un halogène, notamment le chlore, caractérisé en ce que

a) dans une première série de réactions, on fait réagir le composé de départ de formule II

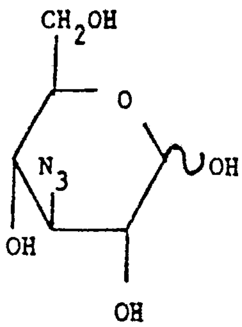

II

sous forme de mélange des anomères α et β avec un agent de glycosylation, constitué par un alcool $R_1OH$, $R_1$ ayant la signification indiquée ci-dessus, pour transformer le groupe OH en position 1, en groupe $OR_1$,

* on traite le produit ainsi obtenu avec un dérivé fonctionnel de l'acide R-COOH pour transformer le groupe OH en position 6, en groupe O-$\underset{\underset{O}{\|}}{C}$-R,

* on sépare les anomères α et β par une méthode classique,

* on fait réagir chacun des anomères α ou β avec un agent d'halogénation pour introduire un halogène en position 4,

le composé obtenu étant sous forme de l'un des anomères α ou β et répondant à la formule III

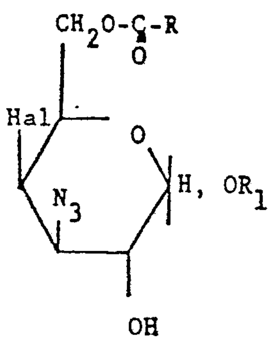

III

dans laquelle

- R et $R_1$ ont les significations indiquées dans la formule I,
- Hal représente un halogène,

b) ensuite, dans une deuxième étape, on effectue la réduction du composé de formule III, pour réduire $N_3$ en $NH_2$, cette réduction étant en outre effectuée dans des conditions telles que l'halogène en position 4 soit ou non réduit en hydrogène, cette réduction étant éventuellement suivie de l'élimination du groupe O-$\underset{\underset{O}{\|}}{C}$-R en position 6 et son remplacement par un groupe OH,

le composé obtenu à l'issue de cette deuxième étape étant sous forme de l'un des anomères α ou β, et répondant à la formule IV suivante :

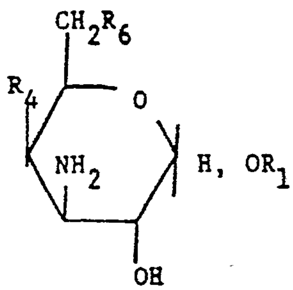

IV

dans laquelle
$R_1$, $R_4$, $R_6$ ont les significations indiquées dans la formule I,
c) enfin, dans une troisième série de réactions, on effectue la réaction des anomères α ou β de formule IV sur un isocyanate d'halogéno éthyle pour transformer $NH_2$ en $NH\underset{\underset{O}{\parallel}}{C}NHCH_2CH_2Hal'$ et on traite le produit ainsi obtenu

par un nitrite de métal alcalin, notamment le nitrite de sodium pour transformer $NH\underset{\underset{O}{\parallel}}{C}NHCH_2CH_2Hal'$ en

$NH\underset{\underset{O}{\parallel}}{C}\underset{\underset{NO}{|}}{N}\ H_2CH_2Hal'$, afin d'obtenir le composé de formule I,
sous forme de l'un des anomères α ou β.

10. Procédé de préparation selon la revendication 9, de dérivés de nitrosourées de formule I caractérisé en ce qu'il comprend

- la réaction du composé de formule II sous forme de mélange des anomères α et β

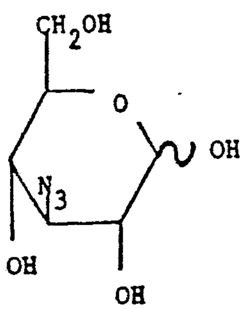

II

avec un agent de glycosylation constitué par $R_1OH$, $R_1$ ayant la signification indiquée à la revendication 9, pour donner le composé de formule V

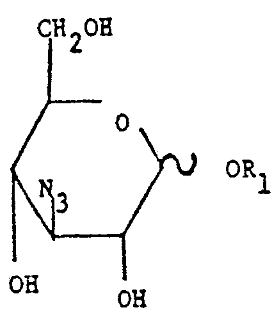

V

- l'introduction d'un groupe acyloxy en position 6, de formule $O-\underset{\underset{O}{\parallel}}{C}-R$, R ayant la signification indiquée à la revendication 9, pour donner le composé de formule

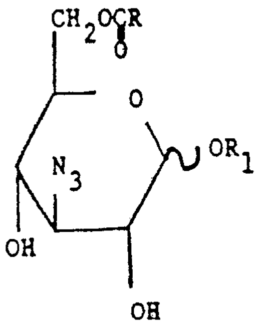

VI

- la séparation du composé VI en deux anomères α et β,

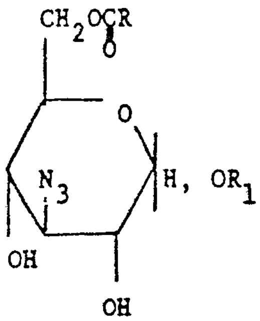

VII

- la réaction des anomères α ou β, de formule VII, avec un agent d'halogénation, pour donner les anomères α ou β de formule III

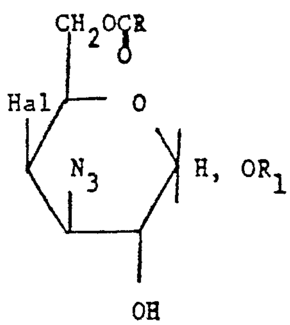

III

. puis

* soit la réduction des anomères α ou β de formule III ainsi obtenus, dans des conditions telles que $N_3$ soit réduit en $NH_2$, sans hydrogénolyse de l'halogène en position 4, pour donner les anomères α ou β de formule IX

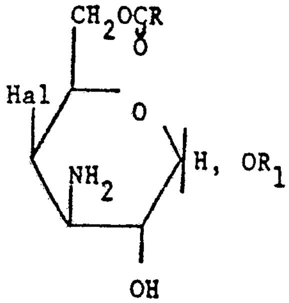

IX

cette réduction étant

** ou bien suivie de la réaction des anomères α ou β de formule IX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$, afin d'obtenir les composés de formule I, dans lesquels $R_4$ représente un halogène et $R_6$ représente $O\text{-}\underset{\underset{O}{\|}}{C}\text{-}R$,

** ou bien suivie de l'élimination à partir des anomères α ou β de formule IX du groupe $O\text{-}\underset{\underset{O}{\|}}{C}\text{-}R$, en position 6, et le remplacement par un groupe OH, puis la réaction des anomères α ou β de formule IV sur un isocyanate d'halogéno éthyle pour transformer $NH_2$ en $NH\ \ NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\text{·}\ H_2CH_2Hal'$,

pour donner les composés de formule I, dans lesquels

$R_4$ représente un halogène et

$R_6$ représente OH,

. ou

* soit la réduction respectivement des anomères III dans des conditions telles que l'halogène en position 4 soit remplacé par H et le groupe $N_3$ soit réduit en $NH_2$, pour donner les anomères α ou β de formule X

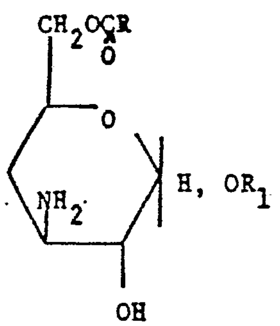

X

cette réduction étant suivie

** ou bien de la réaction des anomères α ou β de formule X sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

respectivement pour obtenir les composés de formule I dans lesquels

$R_4$ représente H,

$R_6$ représente $O\text{-}\underset{\underset{O}{\|}}{C}\text{-}R$

** ou bien de l'élimination à partir des anomères α ou β de formule X du groupe $O\text{-}\underset{\underset{O}{\|}}{C}\text{-}R$ en position 6 et son remplacement par un groupe OH pour donner des anomères α ou β de formule XI

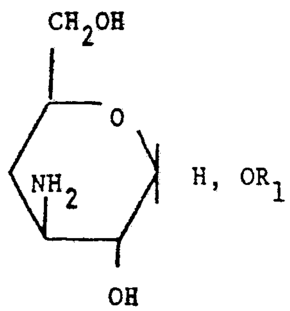

XI

suivi de la réaction des anomères α ou β de formule XI sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$, pour obtenir des anomères α ou β de formule I dans lesquels $R_4$ représente H, et $R_6$ représente OH.

11. Procédé de préparation selon les revendications 9 et 10 de dérivés de nitrosourées de formule I, catactérisé en ce que la réduction des composés de formule III, telle que $N_3$ soit réduit en $NH_2$ et Hal soit remplacé par l'hydrogène, est effectuée à l'aide d'hydrure de tributylétain, en présence d'azo 2-2′ bis isobutyronitrile.

12. Procédé de préparation selon les revendications 9 et 10 de dérivés de nitrosourées de formule I, caractérisé en ce que le composé de formule II est soumis, après la glycosylation, à la réaction de l'oxyde de bis tribulétain et du chlorure de benzoyle, pour introduire le groupe benzoyle en position 6 et obtenir le composé de formule III, dans lequel R représente le groupe phényl sous forme de mélanges d'anomères α et β, ceux-ci pouvant être ensuite séparés.

13. Procédé selon la revendication 9 de préparation de dérivés de nitrosourées de formule I

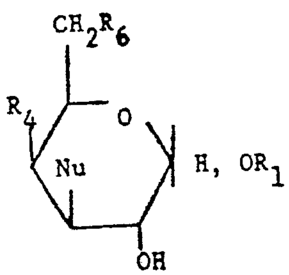

dans laquelle $R_1$, $R_4$, $R_6$ et Nu ont les significations indiquées à la revendication 9, caractérisé en ce qu'il comprend

- la glycosylation du composé de formule II sous forme du mélange d'anomères α et β

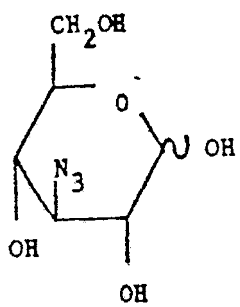

II

à l'aide de $CH_3OH$, pour obtenir le composé de formule XII

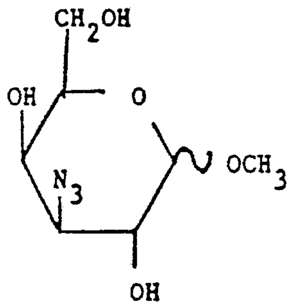

XII

- la réaction du composé de formule XII précédemment obtenu avec du chlorure de benzoyle et de l'oxyde de tributylétain pour obtenir le composé de formule

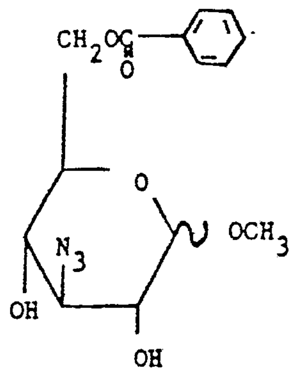

XIII .

- la séparation des deux anomères α et β à partir du composé de formule XIII
- la réaction de l'un ou l'autre des anomères α ou β avec $SO_2Cl_2$, pour obtenir les anomères α ou β de formule

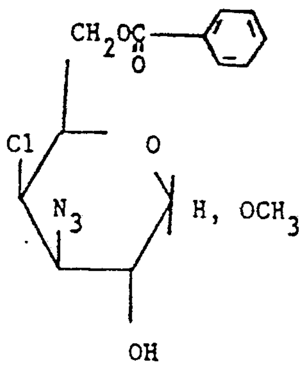

XIV

et

. soit l'hydrogénation catalytique des anomères α ou β de formule XIV précédemment obtenus pour donner les anomères α ou β de formule XV

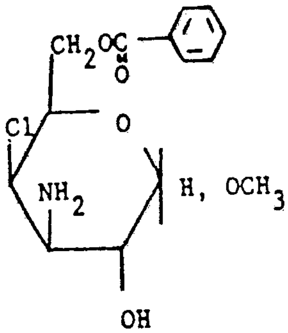

XV

suivi

.. soit de la réaction des anomères α ou β de formule XV sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NC}{|}}{N}H_2CH_2Hal'$,

et pour donner les anomères α ou β de formule

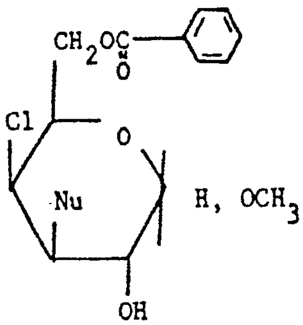

XVI

.. soit de la réaction sur les anomères α ou β de formule XV sur une base, notamment un alcoolate alcalin, pour éliminer le groupe benzoyle et donner les anomères α ou β de formule XVII

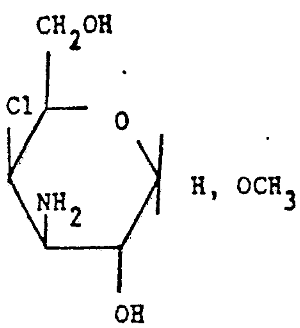

XVII

. suivi de la réaction des anomères α ou β de formule XVII sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$,

et pour donner les anomères α ou β de formule XVIII

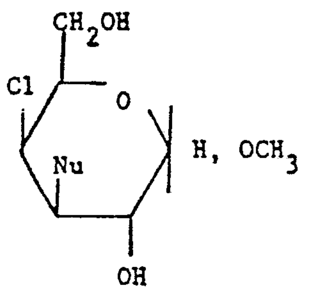

XVIII

. soit la réduction des anomères α ou β de formule XIV, à l'aide d'hydrure de tributylétain, en présence d'azo 2-2' bis isobutyronitrile, pour donner les anomères α ou β de formule XIX

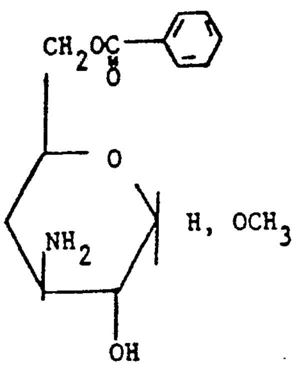

XIX

suivi

.. soit de la réaction des anomères α ou β de formule XIX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium, pour transformer $NH\underset{\underset{O}{\|}}{C}NHCH_2CH_2Hal'$ en $NH\underset{\underset{O}{\|}}{C}\underset{\underset{NO}{|}}{N}\cdot H_2CH_2Hal'$, et pour donner les anomères α ou β de formule XX

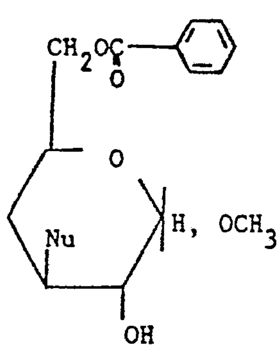

XX

.. soit de la réaction des anomères α ou β de formule XIX avec une base, notamment un alcoolate alcalin, pour éliminer le groupe benzoyle et donner les anomères α ou β de formule XXI

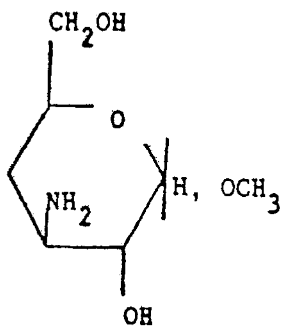

XXI

suivi de la réaction des anomères α ou β de formule XXI sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium, pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$, et pour donner les anomères α ou β de formule XXII

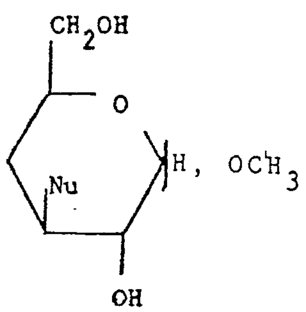

XXII

14. Composés de formule III

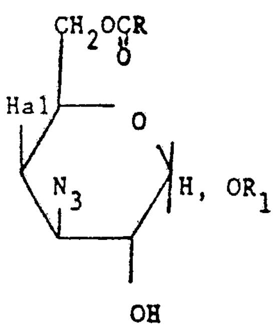

III

dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- R représente un groupe alcoyle de 1 à 6 atomes de carbone, un groupe aryle non substitué ou substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- Hal représente un halogène, notamment le chlore, sous forme de l'un de ses anomères α ou β.

15. Composés selon la revendication 14 de formule III dans laquelle

- $R_1$ représente un groupe alcoyle de 1 à 12 atomes de carbone,

- R représente

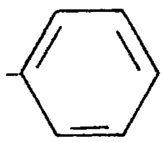

16. Composé de formule

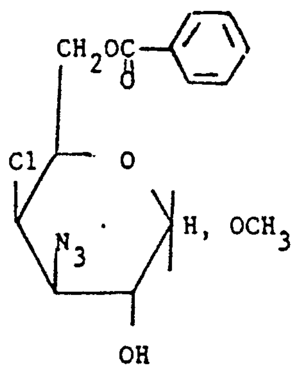

sous forme de l'anomère α ou β.

**Revendications pour l'Etat contractant suivant : ES**

1. Procédé de préparation de dérivés de nitrosourées de formule I

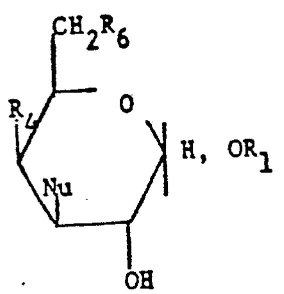

I

sous forme de l'un des deux anomères α ou β,

dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,
- $R_4$ représente Hal, Hal étant un atome d'halogène, notamment Cl,
- $R_6$ représente OH ou $O-\underset{\underset{O}{\|}}{C}-R$,

  R représentant un groupe alcoyle de 1 à 6 atomes de carbone, un groupe aryle non substitué ou substitué sur le noyau aromatique par un ou plusieurs atomes, notamment jusqu'à 3 atomes d'halogène, par un à trois grouped $NO_2$, $CF_3$ ou groupes alcoxy de 1 à 4 atomes de carbone,
- Nu représente le groupe $NH-\underset{\underset{O}{\|}}{C}-\underset{\underset{NO}{|}}{N}-CH_2-CH_2-Hal'$, Hal' identique à Hal ou différent de Hal et représentant un halogène, notamment le chlore, caractérisé en ce que

a) dans une première série de réactions, on fait réagir le composé de départ de formule II

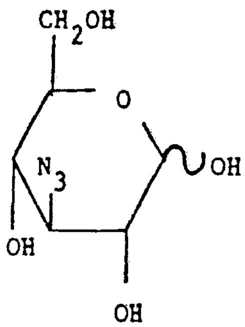

II

sous forme de mélange des anomères α et β avec un agent de glycosylation, constitué par un alcool $R_1OH$, $R_1$ ayant la signification indiquée ci-dessus, pour transformer le groupe OH en position 1, en groupe $OR_1$,

* on traite le produit ainsi obtenu avec un dérivé fonctionnel de l'acide R-COOH pour transformer le groupe OH en position 6, en groupe $O-\underset{\underset{O}{\|}}{C}-R$,

* on sépare les anomères α et β par une méthode classique,

* on fait réagir chacun des anomères α ou β avec un agent d'halogénation pour introduire un halogène en position 4,

le composé obtenu étant sous forme de l'un des anomères α ou β et répondant à la formule III

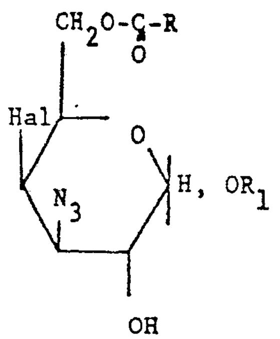

III

dans laquelle

- R et $R_1$ ont les significations indiquées dans la formule I,
- Hal représente un halogène,

b) ensuite, dans une deuxième étape, on effectue la réduction du composé de formule III, pour réduire $N_3$ en $NH_2$, cette réduction étant en outre effectuée dans des conditions telles que l'halogène en position 4 soit ou non réduit en hydrogène, cette réduction étant éventuellement suivie de l'élimination du groupe $O-\underset{\underset{O}{\|}}{C}-R$ en position 6 et son remplacement par un groupe OH,

le composé obtenu à l'issue de cette deuxième étape étant sous forme de l'un des anomères α ou β, et répondant à la formule IV suivante :

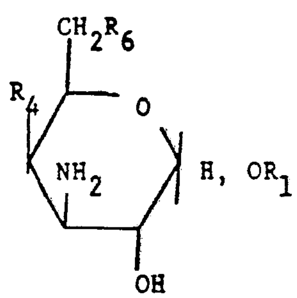

IV

dans laquelle

$R_1$, $R_4$, $R_6$ ont les significations indiquées dans la formule I,

c) enfin, dans une troisième série de réactions, on effectue la réaction des anomères α ou β de formule IV sur un isocyanate d'halogéno éthyle pour transformer $NH_2$ en $NH\underset{\underset{O}{\|}}{C}NHCH_2CH_2Hal'$ et on traite le produit ainsi obtenu

par un nitrite de métal alcalin, notamment le nitrite de sodium pour transformer $NH\underset{\underset{O}{\|}}{C}NHCH_2CH_2Hal'$ en

$NH\underset{\underset{O}{\|}}{C}\underset{\underset{NO}{|}}{N}\ H_2CH_2Hal'$, afin d'obtenir le composé de formule I,

sous forme de l'un des anomères α ou β.

2 . Procédé de préparation selon la revendication 1, de dérivés de nitrosourées de formule I caractérisé en ce qu'il comprend

- la réaction du composé de formule II sous forme de mélange des anomères α et β

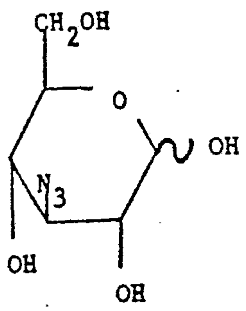

II

avec un agent de glycosylation constitué par $R_1OH$, $R_1$ ayant la signification indiquée à la revendication 9, pour donner le composé de formule V

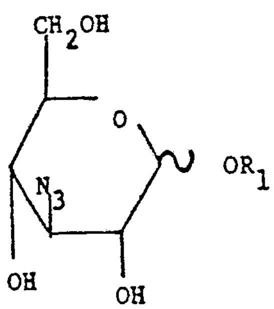

V

- l'introduction d'un groupe acyloxy en position 6, de formule $O\text{-}\underset{\underset{O}{\|}}{C}\text{-}R$, R ayant la signification indiquée à la revendication 9, pour donner le composé de formule

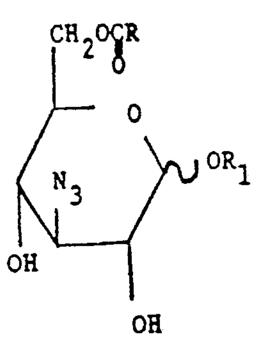

VI

- la séparation du composé VI en deux anomères α et β,

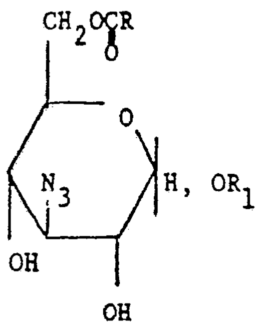

VII

- la réaction des anomères α ou β, de formule VII, avec un agent d'halogénation, pour donner les anomères α ou β de formule III

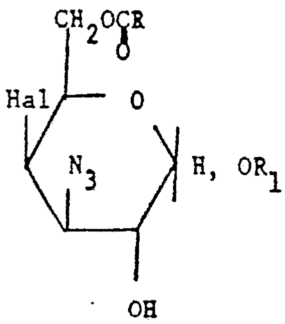

III

. puis

* soit la réduction des anomères α ou β de formule III ainsi obtenus, dans des conditions telles que $N_3$ soit réduit en $NH_2$, sans hydrogénolyse de l'halogène en position 4, pour donner les anomères α ou β de formule IX

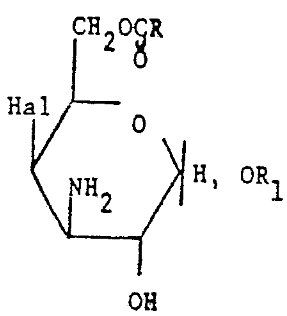

IX

cette réduction étant

** ou bien suivie de la réaction des anomères α ou β de formule IX sur un isocyanate d'halogéno éthyle pour transformer $NH_2$ en $NH\underset{\underset{O}{\|}}{C}NHCH_2CH_2Hal'$ et de

l'action d'un nitrite de métal alcalin, notamment le nitrite de sodium pour transformer $NH\underset{\underset{O}{\|}}{C}NHCH_2CH_2Hal'$ en

$NH\underset{\underset{O}{\|}}{C}\underset{\underset{NO}{|}}{N}H_2CH_2Hal'$, afin d'obtenir les

composés de formule I, dans lesquels $R_4$ représente un halogène et $R_6$ représente $O\text{-}\underset{\underset{O}{\|}}{C}\text{-}R$,

** ou bien suivie de l'élimination à partir des anomères α ou β de formule IX du groupe $O\text{-}\underset{\underset{O}{\|}}{C}\text{-}R$, en position 6, et le

remplacement par un groupe OH, puis la réaction des anomères α ou β de formule IV sur un isocyanate d'halogéno éthyle pour transformer $NH_2$ en $NH\underset{\underset{O}{\|}}{C}NHCH_2CH_2Hal'$ et de l'action d'un

nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\cdot H_2CH_2Hal'$,

pour donner les composés de formule I, dans lesquels

$R_4$ représente un halogène et

$R_6$ représente OH,

. ou

*soit la réduction respectivement des anomères III dans des conditions telles que l'halogène en position 4 soit remplacé par H et le groupe $N_3$ soit réduit en $NH_2$, pour donner les anomères α ou β de formule X

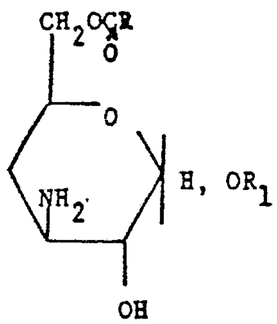

X

cette réduction étant suivie

** ou bien de la réaction des anomères α ou β de formule X sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\cdot H_2CH_2Hal'$,

respectivement pour obtenir les composés de formule I dans lesquels

$R_4$ représente H,

$R_6$ représente $O-\underset{\underset{O}{\|}}{C}-R$

** ou bien de l'élimination à partir des anomères α ou β de formule X du groupe $O-\underset{\underset{O}{\|}}{C}-R$ en position 6 et

son remplacement par un groupe OH pour donner les anomères α ou β de formule XI

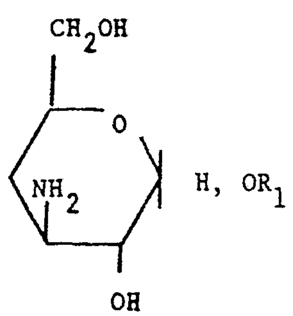

XI

suivi de la réaction des anomères α ou β de formule XI sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

pour obtenir des anomères α ou β de formule I dans lesquels $R_4$ représente H, et $R_6$ représente OH.

3. Procédé de préparation selon les revendications 1 et 2 de dérivés de nitrosourées de formule I, caractérisé en ce que la réduction des composés de formule III, telle que $N_3$ soit réduit en $NH_2$ et Hal soit remplacé par l'hydrogène, est effectuée à l'aide d'hydrure de tributylétain, en présence d'azo 2-2′ bis isobutyronitrile.

4. Procédé de préparation selon les revendications 1 et 2 de dérivés de nitrosourées de formule I, caractérisé en ce que le composé de formule II est soumis, après la glycosylation, à la réaction de l'oxyde de bis tribulétain et du chlorure de benzoyle, pour introduire le groupe benzoyle en position 6 et obtenir le composé de formule III, dans lequel R représente le groupe phényl sous forme de mélanges d'anomères α et β, ceux-ci pouvant être ensuite séparés.

5. Procédé selon la revendication 1 de préparation de dérivés de nitrosourées de formule I

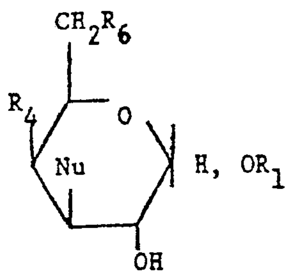

dans laquelle $R_1$, $R_4$, $R_6$ et Nu ont les significations indiquées à la revendication 1, caractérisé en ce qu'il comprend

- la glycosylation du composé de formule II sous forme du mélange d'anomères α et β

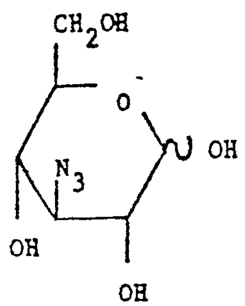

II

à l'aide de $CH_3OH$, pour obtenir le composé de formule XII

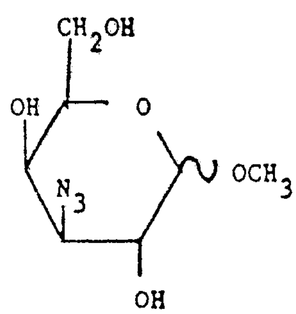

XII

- la réaction du composé de formule XII précédemment obtenu avec du chlorure de benzoyle et de l'oxyde de tributylétain pour obtenir le composé de formule

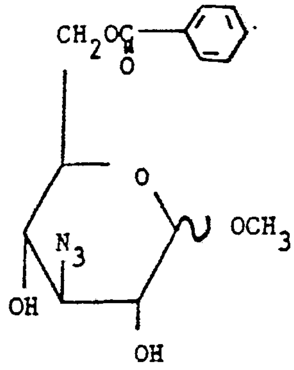

XIII

- la séparation des deux anomères α et β à partir du composé de formule XIII
- la réaction de l'un ou l'autre des anomères α ou β avec $SO_2Cl_2$, pour obtenir les anomères α ou β de formule

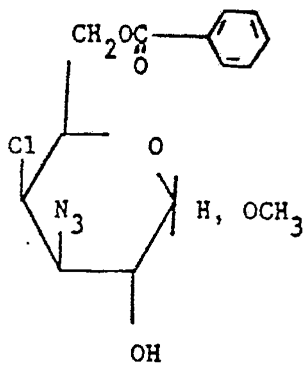

XIV

et

. soit l'hydrogénation catalytique des anomères α ou β de formule XIV précédemment obtenus pour donner les anomères α ou β de formule XV

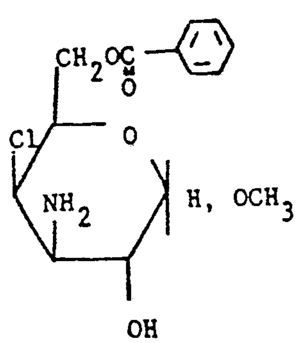

XV

suivi

.. soit de la réaction des anomères α ou β de formule XV sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}H_2CH_2Hal'$,

et pour donner les anomères α ou β de formule

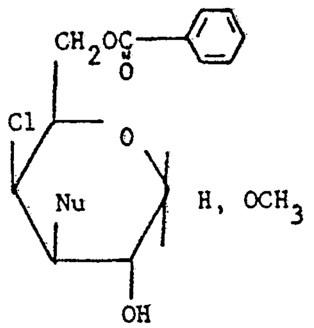

XVI

.. soit de la réaction sur les anomères α ou β de formule XV avec une base, notamment un alcoolate alcalin, pour éliminer le groupe benzoyle et donner les anomères α ou β de formule XVII

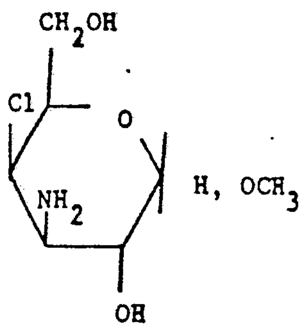

XVII

. suivi de la réaction des anomères α ou β de formule XVII sur un isocyanate d'halogéno éthyle pour transformer $NH_2$ en $NH\underset{\underset{O}{\|}}{C}NHCH_2CH_2Hal'$

et de l'action d'un nitrite de métal alcalin, notamment le nitrite de sodium pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$,

et pour donner les anomères α ou β de formule XVIII

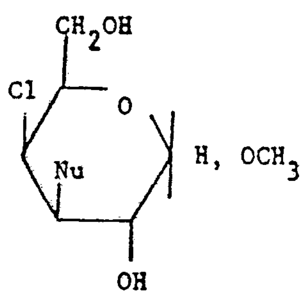

XVIII

. soit la réduction des anomères α ou β de formule XIV, à l'aide d'hydrure de tributylétain, en présence d'azo 2-2′ bis isobutyronitrile, pour donner les anomères α ou β de formule XIX

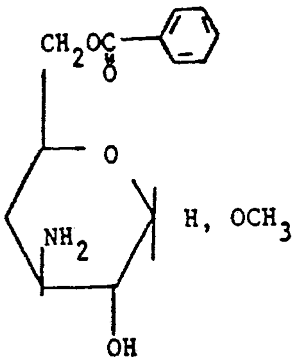

XIX

suivi

.. soit de la réaction des anomères α ou β de formule XIX sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium, pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$, et pour donner les anomères α ou β de formule XX

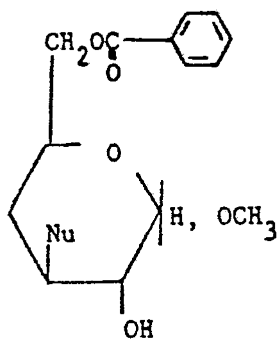

XX

.. soit de la réaction des anomères α ou β de formule XIX avec une base, notamment un alcoolate alcalin, pour éliminer le groupe benzoyle et donner les anomères α ou β de formule XXI

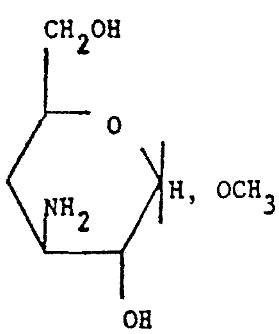

XXI

suivi de la réaction des anomères α ou β de formule XXI sur un isocyanate d'halogéno éthyle pour transformer $NH_2$ en $NH\underset{\underset{O}{\|}}{C}NHCH_2CH_2Hal'$ et de l'action d'un nitrite de métal alcalin, notamment le nitrite de sodium, pour transformer $NH\underset{\underset{O}{\|}}{C}NHCH_2CH_2Hal'$ en $NH\underset{\underset{O}{\|}}{C}\underset{\underset{NO}{|}}{N}\ H_2CH_2Hal'$, et pour donner les anomères α ou β de formule XXII

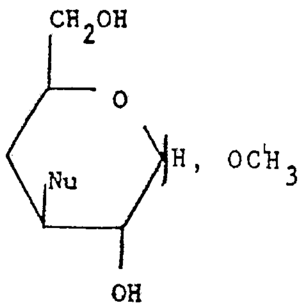

XXII

6. Procédé de préparation, selon la revendication 1, de dérivés de nitrosourées de formule I, dans laquelle
- $R_1$ représente un groupe alcoyle de 1 à 12 atomes de carbone,
- $R_4$ représente un atome d'halogène,
- $R_6$ représente les significations indiquées à la revendication 1.

7. Procédé de préparation, selon la revendication 1, de dérivés de nitrosourées de formule I, dans laquelle
- $R_1$ représente un groupe $CH_3$,
- $R_4$ représente un atome d'halogène,
- $R_6$ représente les significations indiquées à la revendication 1.

8. Procédé de préparation, selon la revendication 1, de dérivés de nitrosourées de formule I, dans laquelle
- $R_1$ représente un groupe $CH_3$,
- $R_4$ représente Cl,
- $R_6$ représente les significations indiquées à la revendication 1.

9. Procédé de préparation, selon la revendication 1, de dérivés de nitrosourées de formule I, dans laquelle
- $R_1$ représente un groupe $CH_3$,
- $R_4$ représente un halogène,
- $R_6$ représente $O\underset{\underset{O}{\|}}{C}R$, R ayant la signification indiquée à la revendication 1.

10. Procédé de préparation, selon la revendication 1, de dérivés de nitrosourées de formule I, dans laquelle
- $R_1$ représente un groupe $CH_3$,
- $R_4$ représente l'hydrogène,
- $R_6$ représente OH.

11. Procédé de préparation, selon la revendication 1, de dérivés de nitrosourées de formule I, dans laquelle
- $R_1$ représente un groupe $CH_3$,
- $R_4$ représente l'hydrogène,
- $R_6$ représente $O-\underset{\underset{O}{\|}}{C}-R$, R ayant la signification indiquée à la revendication 1.

12. Procédé de préparation, selon la revendication 1, de dérivés de nitrosourées de formule I, choisis parmi ceux ayant des formules suivantes

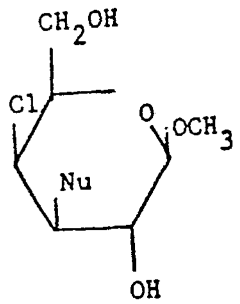

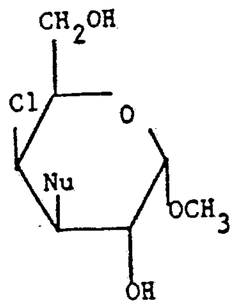

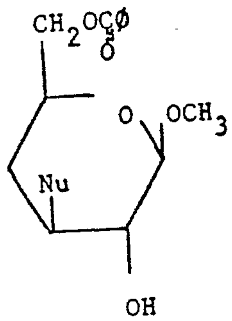

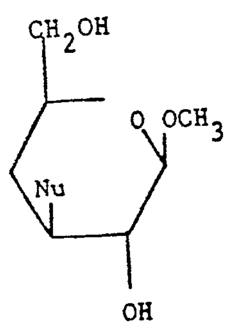

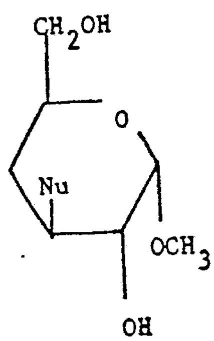

13. Procédé de préparation de composés de formule III :

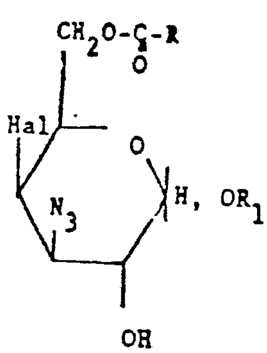

I

sous forme de l'un des deux anomères α ou β,

dans laquelle

- $R_1$ représente un groupe alcoyle de 1 à 12 atomes, ou un groupe aralcoyle de 7 à 12, de préférence de 7 à 9 atomes de carbone, éventuellement substitué sur le noyau aromatique par un ou plusieurs, notamment jusqu'à 3, atomes d'halogène, par un à trois groupes $NO_2$, $CF_3$ ou groupes alcoxy de 1 à 4 atomes de carbone,

- R représente un groupe alcoyle de 1 à 6 atomes de carbone, un groupe aryle non substitué ou substitué sur le noyau aromatique par un ou plusieurs, notoamment jusqu'à 3, atomes d'halogène, par un à trois groupes $NO_2$, $CF_3$ ou groupes alcoxy de 1 à 4 atomes de carbone,
- Hal représente un halogène, notamment le chlore,

caractérisé en ce qu'il comprend :

a) dans une première série de réactions, on fait réagir le composé de départ de formule II

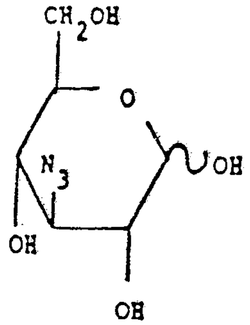

II

sous forme de mélange des anomères α et β avec un agent de glycosylation, constitué par un alcool $R_1OH$, $R_1$ ayant la signification indiquée ci-dessus, pour transformer le groupe OH en position 1, en groupe $OR_1$,

* on traite le produit ainsi obtenu avec un dérivé fonctionnel de l'acide R-COOH pour transformer le groupe OH en position 6, en groupe $O-\underset{\underset{O}{\|}}{C}-R$,

* on sépare les anomères α et β par une méthode classique,

* on fait réagir chacun des anomères α ou β avec un agent d'halogénation pour introduire un halogène en position 4,

le composé obtenu étant sous forme de l'un des anomères α ou β et répondant à la formule III indiquée ci-dessus.

14. Procédé de préparation selon la revendication 13 de composés de formule III caractérisé en ce qu'il comprend

- la réaction du composé de formule II sous forme de mélange des anomères α et β

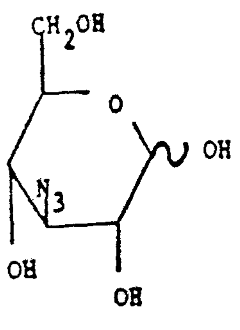

II

avec un agent de glycosylation constitué par $R_1OH$, $R_1$ ayant la signification indiquée à la revendication 4, pour donner le composé de formule V

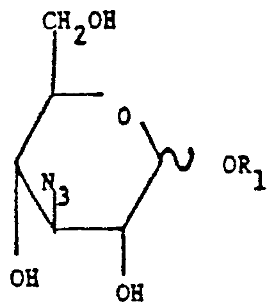

V

- l'introduction d'un groupe acyloxy en position 6, de formule $O-\underset{\overset{\|}{O}}{C}-R$, R ayant la signification indiquée à la revendication 4, pour donner le composé de formule

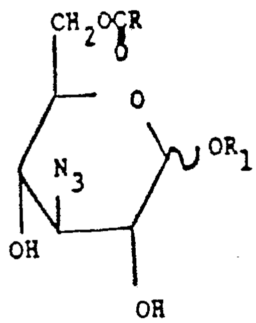

VI

- la séparation du composé VI en deux anomères α et β,

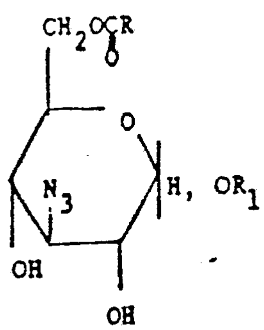

VII

- la réaction des anomères α ou β, de formule VII, avec un agent d'halogénation, pour donner les anomères α ou β de formule III indiquée ci-dessus.

15. Procédé de préparation selon les revendications 13 et 14 de composés de formule III, caractérisé en ce que le composé de formule II est soumis, après la glycosylation, à la réaction de l'oxyde de bis tribulétain et du chlorure de benzoyle, pour introduire le groupe benzoyle en position 6 et obtenir le composé de formule III, dans lequel R représente le groupe phényle, sous forme de mélange d'anomères et , ceux-ci pouvant être ensuite séparés.

16. Procédé selon la revendication 13 de préparation de composés de formule XIV :

XIV 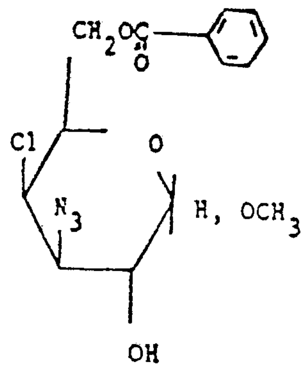

caractérisé en ce qu'il comprend

- la glycosylation du composé de formule II sous forme de mélange d'anomères α et β

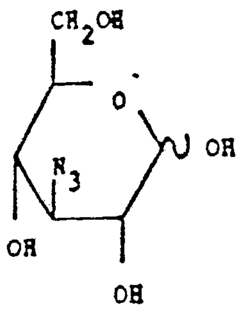 II

à l'aide de $CH_3OH$, pour obtenir le composé de formule XII

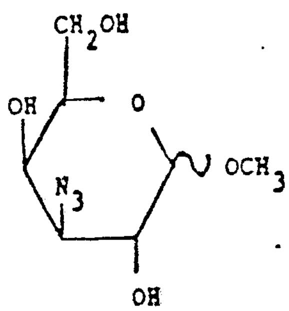 XII

- la réaction du composé de formule XII précédemment obtenu avec du chlorure de benzoyle et de l'oxyde de tributylétain pour obtenir le composé de formule

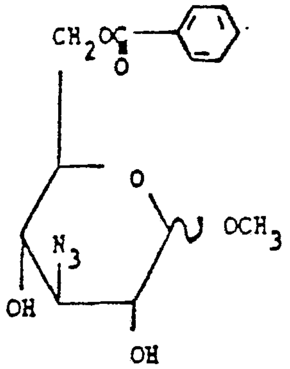

XIII

- la séparation des deux anomères α et β à partir du composé de formule XIII
- la réaction de l'un ou l'autre des anomères α ou β avec $SO_2Cl_2$, pour obtenir les anomères α ou β de formule

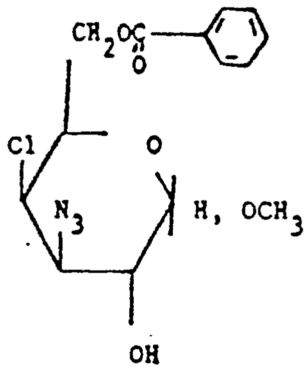

XIV

17. Procédé de préparation d'une composition pharmaceutique, caractérisé en ce que l'un au moins des composés obtenus selon les revendications 1 à 12 sont associés à un véhicule pharmacologiquement acceptable dans des conditions permettant d'obtenir la susdite composition pharmaceutique.